# EUROPEAN PATENT APPLICATION

(11) **EP 3 804 754 A1**
(43) Date of publication of application: **14.04.2021**
(21) Application number: 19306323.7
(22) Date of filing: 09.10.2019
(51) Int. Cl.: A61K 39/395, A61P 29/00, A61P 35/00, C07K 16/28, G01N 33/50

(54) **CMKLR1 AGONISTS HAVING A RESOLVIN E1-LIKE CAPABILITY AND THEIR THERAPEUTIC APPLICATIONS**

(71) Applicant: OSE Immunotherapeutics, 44200 Nantes (FR)
(72) Inventor: GAUTTIER, Vanessa, 44400 Reze (FR); POIRIER, Nicolas, 44119 Treillieres (FR); MARY, Caroline, 44680 Sainte-Pazanne (FR); TRILLEAUD, Charlène, 44400 Rèze (FR)
(74) Representative: Ernest Gutmann - Yves Plasseraud S.A.S.

(57) **Abstract**

The present invention provides anti-chemerin receptor antibodies which have a resolvin E1-like agonist activity on chemokine like receptor-1 (CMKLR1) for use in the resolution of an inflammation occurring during autoimmune diseases, chronic inflammatory diseases, infectious diseases, cancers, by sustaining or initiating the resolution phase of inflammation.

## Description

### Field of the invention

The invention pertains to the field of immunotherapy. The present invention provides anti-chemerin receptor antibodies which have a resolvin E1-like agonist activity on chemokine like receptor-1 (CMKLR1 or ChemR23), for use in the resolution of an inflammation in particular occurring during cancer, an autoimmune disease, an infection, a chronic inflammatory disease, a chronic infection or sepsis, by sustaining or initiating the resolution phase of inflammation.

### Background of the invention

The critical role of inflammatory processes in health and diseases has long been recognized. The detailed molecular mechanisms and biological events that regulate the progression and the resolution of inflammation remain of critical interest. Recent investigations have provided strong evidence that the resolution of inflammation is not a passive process, as believed earlier. Resolution of the inflammation is instead a biosynthetically active process, regulated by biochemical mediators and receptors-signaling pathways. Resolution is therefore driven by specialized pro-resolving mediators. Inflammation is a physiological mechanism that occurs during an infection, an injury or a traumatism. Inflammation is inevitable and usually salutary, and its response is orchestrated by a delicate balance between positive and negative feedback loops. The inflammation is usually divided in 3 steps: initiation, amplification and resolution.

The resolution process which allows the ending of the inflammatory response is a complex process involving the sequential and chronological engagement of cellular (e.g. granulocytes or macrophages) and chemical (e.g. cytokines or specialized pro-solving mediators or factors) effectors.

Chemokine-like receptor 1 (CMKLR1), also known as ChemR23, and chemokine receptor-like 2 (CCRL2) are 7-transmembrane receptors identified by their homology to known G-protein-coupled receptors (AJ Kennedy and AP Davenport, 2018). The Chemokine-like Receptor 1 (CMKLR1; also named Dez in Murine animals), is an orphan G protein-coupled receptor related to GPR-1 (38% overall amino acid identity), C3a receptor (38%), C5a anaphylatoxin receptor (36%) and formyl Met-Leu-Phe receptors (35%). CMKLR1 is more distantly related to the chemokine receptors subfamily (Samson et al., 1998). CMKLR1 is expressed on monocytes, on macrophages, on dendritic cells, and NK on cells, as well as on adipocytes and endothelial cells. At least two ligands have been identified. The first ligand is the protein Chemerin, which is a chemoattractant protein exerting a stimulation of dendritic cells and macrophages to the site of inflammation.

The second ligand of CMKLR1 is the lipid mediator Resolvin E1 (RvE1 - Formal Name: 5S,12R,18R-trihydroxy-6Z,8E,10E,14Z,16E-eicosapentaenoic acid; CAS Number: 552830-51-0) that belongs to the Resolvin family. The anti-inflammatory lipid mediator Resolvin E1 inhibits leukocyte infiltration and pro-inflammatory gene expression.

Initially, the interest in the chemerin system (*i.e*. the signaling pathway(s) activated or not by Chemerin receptors by their ligands like Chemerin and Resolvin) was focused on its role in inflammation and chemotaxis of immune cells following its discovery in psoriasis disease. Most recently, in connection with its role in inflammation, in obesity, metabolic syndrome, it's potential role in association with cardiovascular functions has been considered, as well as role in reproductive biology. Therefore, the chemerin system is of major interest for its role in the inflammation process, in particular for its role in the resolution of the inflammation. A number of diseases are related to delay or disruption of the resolution process. Most of the specialized pro-resolving factor mediators currently known are derived from polyunsaturated fatty acids, including lipoxins, the resolving family, including E-series resolvins and D-series resolvins, protectins, and maresins. Nonetheless, pro-resolving molecules are difficult to synthesize because of their lipidic nature. Production of pro-resolving molecules in sufficient quantities, for a clinical trial for example, is a burden, and very few SPM have gone through efficient production. Besides that, antibodies specifically targeting G-protein-coupled receptors are difficult to produce. There is therefore a need for molecules having the capability to take part, in particular to initiate or enhance the resolution stage of the inflammatory response like pro-resolving factors.

A number of diseases are related to delay or disruption of the resolution process. Most of the specialized pro-resolving factor mediators currently known are derived from polyunsaturated fatty acids, including lipoxins, the resolving family, including E-series resolvins and D-series resolvins, protectins, and maresins. Nonetheless, pro-resolving molecules are difficult to synthesize because of their lipidic nature. Production of pro-resolving molecules in sufficient quantities, for a clinical trial for example, is a burden, and very few SPM have gone through efficient production. Besides that, antibodies specifically targeting G-protein-coupled receptors are difficult to produce. There is therefore a need for molecules having the capability to take part, in particular to initiate or enhance the resolution stage of the inflammatory response like pro-resolving factors.

The present inventors showed for the first time that the use of an agonist of CMKLR1 having a Resolvin E1-like capability induces positive effects towards the resolution of the inflammation. As illustrated in the present description of the invention, the CMKLR1-agonist compound of the invention exerts various effects in myeloid cells, leading to initiation and/or progression of the resolution stage of an inflammatory process. In the following description, without any contrary mention, agonist of CMKLR1 having a Resolvin E1-like capability may be identified as the or a "agonist"; both terms include an antibody or antigen-bind fragment thereof (also reference anti-CMKLR1 antibody), a protein, a peptide or a polypeptide; the term compound or anti-CMKLR1 compound may also be used in the following description as a synonym of "agonist" (of the invention), thereby including an antibody or antigen-bind fragment thereof (also reference anti-CMKLR1 antibody), a protein, a peptide or a polypeptide. Among the effects provided by the use of such an agonist, the following particular effects have been demonstrated:
- an agonist of CMKLR1 having a Resolvin E1-like capability induces the apoptosis of polymorphonuclear neutrophils (also simply referenced neutrophils, PMN or PMNs herein), and/or the decrease or the inhibition of the migration capability of these cells, in particular through the inhibition of their capacity of transmigration through endothelium towards the site of inflammation as described later in the application
- an agonist of CMKLR1 having a Resolvin E1-like capability induces the internalization of different receptors expressed on the cell surface on various myeloid cells, notably macrophages and/or dendritic cells, thereby enhancing the processes which induce or sustain the resolution of the inflammation as described later in the application;
- in particular an agonist of CMKLR1 having a Resolvin E1-like capability induces the internalization of different receptors CMKLR1, and CXCR4 and/or CCR7 expressed on the cell surface of macrophages and/or dendritic cells, such internalization leading to the much lower targeting and recognition of the CXCR4, CCR7 receptors by cytokines known to induce the migration of the cells in direction to the site of inflammation, and as a consequence leading to the decrease or inhibition of the migration of macrophages and/or dendritic cells from the site of inflammation to secondary lymphoid organs and/or towards the site of inflammation;
- An agonist of CMKLR1 having a Resolvin E1-like capability decreases or inhibits the capability of neutrophils and macrophages and/or dendritic cells to migrate; in particular, it decreases or inhibits the ability of neutrophils to transmigrate through the site of inflammation by lowering the rolling capability of these cells due to the internalization and/or the decrease of cell surface expression of CD62L, reducing their ability to transmigrate through the endothelium;
- In a particular aspect of the invention, the inventors demonstrated that agonist of CMKLR1 having a Resolvin E1-like capability comprising a domain suitable for interaction with a Fc receptor, like IgG constant domain, in particular IgG1 constant domain, are particularly efficient. The Fc Receptors of macrophages or neutrophils recognize particularly very efficiently the Fc fragment of the anti-CMKLR1 IgG-constant domain, or IgG1 constant domain, leading or contributing to the apoptosis of the neutrophils recognized by the anti-CMKLR1 IgG1-antibodies
- The inventors herein demonstrate that myeloid cells which are involved in the inflammation process (*i.e*. which sustain inflammation) and which express CMKLR1, and CXCR4 and/or CCR7, are particularly suitable targets for an agonist of CMKLR1 having a Resolvin E1-like capability, for the treatment of a pathological inflammation process.

Therefore, agonists of CMKLR1 having a Resolvin E1-like capability represent a useful therapeutic tool to enhance or sustain the resolution of the inflammation, and/or inhibit negative effects of an inflammation, in particular in chronic inflammation. Some chemical agents have proven their pro-resolutive effect on acute inflammation but none of them is able to positively induce or sustain the resolution of a chronic inflammation.

### Summary of the invention

The invention relates to an agonist of CMKLR1 having a Resolvin E1-like capability for use in the therapeutic treatment of an inflammatory condition in a patient, in particular an inflammatory condition wherein the resolution phase of the inflammation is delayed or disrupted, in particular in a chronic inflammation, in particular said agonist being selected from the group consisting of an antibody or antigen-binding fragment thereof, a peptide, a polypeptide and a protein.

In a first aspect, the invention relates to an agonist of anti-Chemerin Like Receptor 1 (CMKLR1) having a Resolvin E1-like capability, for use in the treatment of a patient suffering from an inflammatory condition, in particular an inflammatory condition wherein the resolution of inflammation is delayed or disrupted, wherein the agonist of CMKLR1 having a Resolvin E1-like capability is selected from the group consisting of an antibody or antigen-binding fragment thereof, a peptide, a polypeptide and a protein, and wherein the agonist:
- induces or activates the apoptosis of neutrophils at the site of inflammation, and/or
- inhibits or decreases the transmigration capability of neutrophils through endothelium towards the site of inflammation, and/or
- inhibits the migration of macrophages and/or of dendritic cells from the site of inflammation to secondary lymphoid organs and/or towards the site of inflammation. The invention relates to the use of an agonist of CMKLR1 having a Resolvin E1-like capability, like an antibody, in particular a humanized antibody, or antigen-binding fragment thereof, to induce or enhance or activate the apoptosis of neutrophils, in the treatment of an inflammatory condition, in particular an inflammatory condition wherein the resolution of inflammation is delayed or disrupted.

In a second aspect, the invention relates to an agonist of CMKLR1 having a Resolvin E1-like capability in particular an antibody or a humanized antibody, or antigen-binding fragment thereof, for use in the treatment of an inflammatory condition, in particular an inflammatory condition wherein the resolution of inflammation is delayed or disrupted, wherein the condition may be improved or prevented by reducing or inhibiting the transmigration capability of neutrophils to a site of inflammation.

The invention relates to the use of an agonist of CMKLR1 having a Resolvin E1-like capability, in particular an antibody or a humanized antibody, or antigen-binding fragment thereof, to reduce or inhibit the transmigration capability of neutrophils to a site of inflammation, for the treatment of an inflammatory condition, in particular an inflammatory condition wherein the resolution of inflammation is delayed or disrupted.

In a particular aspect, the invention relates to an agonist of CMKLR1 having a Resolvin E1-like capability, in particular an antibody or a humanized antibody, or antigen-binding fragment thereof, to reduce or inhibit the capability of macrophages to migrate from a site of inflammation to secondary lymphoid organs, for use in the treatment of an inflammatory condition, in particular an inflammatory condition wherein the resolution of inflammation is delayed or disrupted.

In a particular aspect, the invention relates to an agonist of CMKLR1 having a Resolvin E1-like capability, in particular an antibody or humanized antibody, or antigen-binding fragment thereof, for use in the treatment of an inflammatory condition, in particular an inflammatory condition wherein the resolution of inflammation is delayed or disrupted, wherein the condition may be improved by decreasing the cell surface expression of CMKLR1 and CXCR4 and/or CCR7. In a particular embodiment, the cell surface expression is considered on macrophages and/or dendritic cells.

In a particular aspect, the invention relates to an agonist of CMKLR1 having a Resolvin E1-like capability, in particular an antibody or a humanized antibody, or antigen-binding fragment thereof, to decrease the cell surface expression of CMKLR1, and CXCR4 and/or CCR7, for use in the treatment of an inflammatory condition, in particular an inflammatory condition wherein the resolution of inflammation is delayed or disrupted. In a particular embodiment, the cell surface expression is considered on macrophages and/or dendritic cells.

In a particular aspect, the invention relates to an agonist of CMKLR1 having a Resolvin E1-like capability y, in particular an antibody or a humanized antibody, or antigen-binding fragment thereof, for use in the treatment of an inflammatory condition, in particular an inflammatory condition wherein the resolution of inflammation is delayed or disrupted, wherein the condition may be improved by inducing the internalization of CMKLR1, and CXCR4 and/or CCR7. In a particular embodiment, the internalization is considered on macrophages and/or dendritic cells.

In a particular aspect, the invention relates to an agonist of CMKLR1 having a Resolvin E1-like capability, in particular an antibody or a humanized antibody, or antigen-binding fragment thereof, to induce the internalization of CMKLR1, and CXCR4 and/or CCR7, for use in the treatment of an inflammatory condition, in particular an inflammatory condition wherein the resolution of inflammation is delayed or disrupted. In a particular embodiment, the internalization is considered on macrophages and/or dendritic cells.

In a particular aspect, the invention relates to an agonist of CMKLR1 having a Resolvin E1-like capability, in particular an antibody or a humanized antibody, or antigen-binding fragment thereof, for use in the treatment of an inflammatory condition, in particular an inflammatory condition wherein the resolution of inflammation is delayed or disrupted, wherein the condition may be improved by decreasing the cell surface expression/recognition of CD62L. In a particular embodiment, the cell surface expression is considered on neutrophils.

In a particular aspect, the invention relates to an agonist of CMKLR1 having a Resolvin E1-like capability, in particular an antibody or a humanized antibody, or antigen-binding fragment thereof, to decrease the cell surface expression/recognition of CD62L, for use in the treatment of an inflammatory condition, in particular an inflammatory condition wherein the resolution of inflammation is delayed or disrupted. In a particular embodiment, the cell surface expression is considered on neutrophils.

In a particular aspect, the invention relates to an agonist of CMKLR1 having a Resolvin E1-like capability, in particular an antibody or a humanized antibody, or antigen-binding fragment thereof, for use in the treatment of an inflammatory condition, in particular an inflammatory condition wherein the resolution of inflammation is delayed or disrupted, wherein the condition may be improved by inducing the internalization/or shedding of CD62L. In a particular embodiment, the internalization and/orshedding is considered on neutrophils.

In a more particular embodiment, the agonist of CMKLR1 having a Resolvin E1-like capability is an antibody which is or is issued or is derived from a human IgG1 isotype.

In a particular aspect, the invention relates to an agonist of CMKLR1 having a Resolvin E1-like capability, in particular an antibody or a humanized antibody, or antigen-binding fragment thereof, to induce the internalization of CD62L, for use in the treatment of an inflammatory condition, in particular an inflammatory condition wherein the resolution of inflammation is delayed or disrupted. In a particular embodiment, the internalization is considered on neutrophils. In a more particular embodiment, the agonist of CMKLR1 having a Resolvin E1-like capability is an antibody which is or is issued or is derived from a human IgG1 isotype.

In a particular aspect, the invention also relates to a method for treating an inflammatory condition, in particular an inflammatory condition wherein the resolution of inflammation is delayed or disrupted by administering an agonist of CMKLR1 having a Resolvin E1-like capability.

In a particular aspect, the invention relates to a method for treating an inflammatory condition, in particular an inflammatory condition wherein the resolution of inflammation is delayed or disrupted, by inducing or enhancing or activating the apoptosis of neutrophils by administering an agonist of CMKLR1 having a Resolvin E1-like capability.

In a particular aspect, the invention relates to a method for treating an inflammatory condition, in particular an inflammatory condition wherein the resolution of inflammation is delayed or disrupted, by reducing or inhibiting the transmigration capability of neutrophils to a site of inflammation by administering an agonist of CMKLR1 having a Resolvin E1-like capability.

In a particular aspect, the invention relates to a method for treating an inflammatory condition, in particular an inflammatory condition wherein the resolution of inflammation is delayed or disrupted, by inducing the internalization and/or decrease of CMKLR1 and CXCR4 and/or CCR7 by administering an agonist of CMKLR1 having a Resolvin E1-like capability. In a more particular embodiment, the internalization/decrease is considered on macrophages.

In a particualr embodiment of the invention, the CMKLR1 is a human CMKLR1, which corresponds to NCBI Protein accession number Q99788.2.

### Detailed description of the invention

The invention concerns an agonist of CMKLR1 having a Resolvin E1-like capability, like but not limited to an antibody, for use in the treatment of a pathological condition wherein inflammation is concerned. In the following description, an agonist of CMKLR1 having a Resolvin E1-like capability is an anti-CMKLR1 antibody, or antigen-binding fragment thereof, peptide, polypeptide or protein which binds to CMKLR1 (also known as Chemerin Like Receptor 1 or ChemR23) and has an agonist capability as compared to the binding of Resolvin E1 to CMKLR1; i.e. the agonist has an effect on CMKLR1-positive cells comparable to at least one effect provided by the binding on these cells of resolvin E1. In particular embodiment, the compound may be considered as an anti-CMKLR1 agonist having a Resolvin E1 -like capability when the G-protein signaling pathway is activated in CMKLR1-potitive cells stimulated with the agonist of the invention, and in a more particular embodiment, also when the B-arrestin pathway is not activated, and in particular conditions, the B-arrestin pathway is inhibited In particular, the invention concerns an agonist of CMKLR1 having a Resolvin E1-like capability for use in the treatment of a pathological condition wherein the resolution of the inflammation is delayed or disrupted. "a delay or a disruption in the resolution of an inflammatory condition" occurs when the resolution of inflammation is delayed or disrupted as compared to a normal resolution (i.e. the resolution occurring in a patient who experiences normal resolution after an inflammatory event). A resolution delay or defect can result in an increased penetration of granulocytes at the inflammatory site. Hence, a resolution delay or defect may be assessed by quantification of granulocytes at the inflammatory site. Granulocyte population may be measured for example by histology, cytometry or indirect biochemical techniques such as elastase quantification by enzyme immunoassay or molecular quantification by PCR of granulocyte receptor 1). A resolution delay or defect may also be assessed by the determination of a delay in the apoptosis of granulocytes, measured for example by cytology using specific antibodies against annexin 5. A defect or a delay in the resolution of the inflammation may also be determined by assessing by quantifying the synthesis of pro-inflammatory cytokines such as TNF-alpha, IL8 or IL12 and anti-inflammatory cytokines such as IL-10. Cytokine secretion may be assessed by enzyme immunoassay or by PCR. A defect or a delay in the resolution of the inflammation may also be determined by assessing the activation of transcription factors involved in the synthesis of inflammatory cytokines, such as NF-kappaB which can be measured for example by nuclear translocation or by Western blot and/or by quantification of the level of degradation of IkappaB). A defect or a delay in the resolution of the inflammation may also be determined by quantifying specialized pro-resolving mediators (such as lipoxins, resolvins, protectins or maresins) or their precursors (like 17-HDOHE or 14-HDOHE) by mass spectrometry or enzyme immunoassay. A defect or a delay of the resolution then results in a defect of the synthesis of one or more of these mediators. A resolution defect or delay can also be determined when expression of the receptors of the resolution molecules is decreased. These receptors may be selected from the group comprising ALX, CMK1R1, GPR32 or GPR18. Alternatively or complementarily, the internalization and processing of those receptors into the cytoplasm may also be assessed. Alternatively or complementarily, expression of some receptors of inflammatory cytokines or lipids may also be assessed, an overexpression compared to a normal condition being significant of a delay or a defect in the resolution of the inflammation. These conditions may be measured by histology, cytology or PCR. The resolution defect can also result in a decreased or inhibited switch of proinflammatory to proresolutive macrophages, a damage in phagocytosis or efferocytosis of the same cells. Hence, a delay or a defect of the resolution may be assessed by analyzing the switch of proinflammatory to proresolutive macrophages in a particular condition as compared to a normal condition, as exemplified in the examples of the present invention.

In a preferred embodiment of the invention, the agonist is an antibody, or an antigen-binding fragment-thereof.

An "agonist of CMKLR1 having a Resolvin E1-like capability" may also be defined as an antibody or antigen-binding fragment thereof, a peptide, a polypeptide or protein, able to bind CMKLR1, and thereby able to induce the phosphorylation of the Akt and/or Erk protein(s), as compared to a control antibody. A control antibody may be an antibody which does not specifically bind CMKLR1. Human CMKLR1 corresponds to the amino acid sequence of SEQ ID No. 1. Phosphorylation of a protein may be determined according to methods well known by the skilled artisan, for example by the method disclosed in the examples of the present description. In a particular embodiment, a compound of the invention is a RvE1-like agonist, i.e. a compound of the invention is an agonist of CMKLR1 signaling pathway induced by RvE1. In other words, the anti-CMKLR1 compound of the invention is an agonist of the interaction between RvE1 and CMKLR1, in particular between human RvE1 and human CMKLR1. In a particular embodiment, the compound of the invention enhances activation of the G protein pathway induced by CMKLR1. In another embodiment, the compound of the invention does not induce the activation of the β-arrestin pathway induced by CMKLR1. In another embodiment, the compound of the invention inhibits the β-arrestin pathway induced by CMKLR1. In another embodiment, since a compound of the invention induces at least one agonist effect of the binding of RvE1 to CMKLR1, and since RvE1 is a pro-resolution factor or pro-resolution mediator, a compound of the invention is a pro-resolution factor or a pro-resolution mediator.

As a result of binding and activation of the receptor to produce a biological response, the compounds of the invention may lead to the activation of the G protein signaling pathway, in particular Gαᵢ signaling pathway and/or Gαₒ, without activating the β-arrestin pathway, in particular the compound of the invention may lead to the inhibition of the β-arrestin pathway. In particular, the binding of a compound according to the invention induces the activation of Akt and/or Erk protein(s) *in vitro* and/or *in vivo.* In other words, a Resolvin-E1 like agonist antibody may be defined as an antibody able to bind CMKLR1, and thereby able to induce the phosphorylation of the Akt and/or Erk protein(s), as compared to a control antibody. A control antibody may be an antibody which does not specifically bind CMKLR1. Phosphorylation of a protein may be determined according to methods well known by the skilled artisan, for example by the method disclosed in the examples of the present description. In another embodiment, the compound of the invention does not induce the activation of the β-arrestin pathway induced by CMKLR1. In another embodiment, the compound of the invention inhibits the β-arrestin pathway induced by CMKLR1. In another embodiment, since a compound of the invention induces at least one agonist effect of the binding of RvE1 to CMKLR1, and since RvE1 is a pro-resolution factor or pro-resolution mediator, a compound of the invention is a pro-resolution factor or a pro-resolution mediator.

In a preferred embodiment of the invention, the agonist of CMKLR1 having a Resolvin E1-like capability binds specifically to the third extra loop of CMKLR1 which corresponds to the amino acid sequence of SEQ ID No. 2, and more particularly to an epitope localized within the third extra-loop of CMKLR1, in particular to an epitope located within amino acid residue sequences set forth in SEQ ID No: 2 or consisting of SEQ ID No: 3 or consisting of SEQ ID No. 4. An anti-CMKLR1 compound binding within this particular region of CMKLR1 may have an agonist property on CMKLR1, thereby mimicking the binding of RvE1 to CMKLR1.

It should be noted that, on the contrary to a resolvin mimetic, the provision of an agonist of CMKLR1 having a Resolvin E1-like capability as defined herein provides an interesting pharmacokinetic profile, as well as a long-lasting effects due its better half-life, which is advantageous when the condition to be treated is related to a chronic inflammatory condition.

In a particular embodiment, the agonist of CMKLR1 having a Resolvin E1-like capability has the capability *in vitro* and/or *in vivo* to induce the phosphorylation of Akt and/or Erk after activation of CMKLR1, in particular the phosphorylation of both Akt and Erk induced after activation of CMKLR1, in particular in myeloid cells, and more particularly in macrophages. The antibody is able to induce the phosphorylation of the Akt and/or Erk protein(s), as compared to a control antibody. Phosphorylation of a protein may be determined according to methods well known by the skilled artisan.

In some embodiments, the antibody is a humanized antibody, in particular a humanized monoclonal antibody. As used herein, "humanized" describes antibodies issued from non-human species whose protein sequences have been modified to increase their identity to antibody variants produced naturally in human. Accordingly having been originally obtained in animals, especially in rodents and in particular in rats, following immunization of animals and production of antibodies, particularly monoclonal antibodies from hybridoma, the antibodies are then modified in their VH and/or VL sequences by substitution of amino acid residues, in the framework and optionally in addition in the CDR sequences to obtain humanized antibodies. Particularly, said humanized antibody has less than 10% of mutated amino acid residues, preferably one or no mutated amino acid residue, in individually considered CDR regions with respect to the original CDRs regions. Methods of humanization include, but are not limited to, those described in U.S. Pat. Nos. 4,816,567, 5,225,539, 5,585,089, 5,693,761, 5,693,762 and 5,859,205.

In some embodiments, the antibody is a fully human antibody, in particular a fully human monoclonal antibody. Fully human monoclonal antibodies also can be prepared by immunizing mice transgenic for large portions of human immunoglobulin heavy and light chain loci. See, e.g., U.S. Pat. Nos. 5,591,669, 5,598,369, 5,545,806, 5,545,807, 6,150,584, and references cited therein. These animals have been genetically modified such that there is a functional deletion in the production of endogenous (e.g., murine) antibodies. The animals are further modified to contain all or a portion of the human germ-line immunoglobulin gene locus such that immunization of these animals will result in the production of fully human antibodies to the antigen of interest. Following immunization of these mice (e.g., XenoMouse(Abgenix), HuMAb mice (Medarex/GenPharm)), monoclonal antibodies can be prepared according to standard hybridoma technology. These monoclonal antibodies will have human immunoglobulin amino acid sequences and therefore will not provoke human anti-mouse antibody (KAMA) responses when administered to humans. In vitro methods also exist for producing human antibodies. These include phage display technology (U.S. Pat. Nos. 5,565,332 and 5,573,905) and in vitro stimulation of human B cells (U.S. Pat. Nos. 5,229,275 and 5,567,610).

In a particular embodiment, a compound of the invention does not interfere with the binding of Chemerin to CMKLR1. Chemerin is one of the natural ligand of CMKLR1. In other words, a compound according to the invention is not an agonist nor an antagonist of the interaction between Chemerin and CMKLR1.

The various antibody molecules and fragments may derive from any of the commonly known immunoglobulin classes (isotypes), including but not limited to IgA, secretory IgA, IgE, IgG and IgM. IgG subclasses are also well known to those in the art and include but are not limited to human IgGI, IgG2, IgG3 and IgG4.

The inventors assessed the effect of an agonist of CMKLR1 having a Resolvin E1-like capability in different myeloid cells and demonstrated for the first time that the use of such agonist compound increases the apoptosis of polymorphonuclear neutrophils, referenced herein under the acronym PMN or PMNs, on the inflammation site. PMN cells are a category of blood cells known to contribute to the inflammatory response. Neutrophils are usually located at the site of inflammation and their presence sustain the inflammatory process, thereby preventing the resolution of the inflammation to be initiated or to be actively sustained, which may lead to chronic inflammation. Neutrophils are the most abundant cell type involved in the innate immune response. They are rapidly recruited to sites of injury or infection where they engulf and kill invading microorganisms. Neutrophil apoptosis, the process of programmed cell death that prevents the release of neutrophil histotoxic contents, is tightly regulated and limits the destructive capacity of neutrophil products to surrounding tissue. The induction of apoptosis of neutrophil itself exerts an anti-inflammatory effect through modulation of surrounding cell responses, particularly macrophage phagocytosis. The regulation of apoptosis of neutrophils is crucial for therapeutic intervention in inflammatory related-diseases. As illustrated in the examples of the invention, the use of an agonist of CMKLR1 having a Resolvin E1-like capability on PMNs induces a strong apoptosis of neutrophils, as compared to a control compound, like an antibody, during the inflammation induction. The use of an agonist of CMKLR1 having a Resolvin E1-like capability may therefore lead to reduce the adverse effects of inflammation, especially when its resolution is delayed or disrupted. It should be noted that, in a particular embodiment of the invention, the effect on the apoptosis of neutrophils is not accompanied by any other irreversible negative effect on immune cells, in particular on immune cells not located on the site of inflammation. Except the induced apoptosis of neutrophils on the site of inflammation, a treatment with the compound of the invention does not lead to the apoptosis of all neutrophil population. This feature may be very advantageous to reduce side-effect(s) of a therapy with the compounds described herein. The effect on neutrophils apoptosis may therefore be limited, *i.e*. the apoptosis of neutrophils may be limited to a portion of the overall population of neutrophils, thereby limiting the side effects associated with depletion of neutrophils, but having a sufficient effect to induce or sustain the resolution of the inflammation. Accordingly, in a preferred embodiment of the invention, the agonist of CMKLR1 having a Resolvin E1-like capability induces a limited apoptosis of neutrophils, as compared to a control, between 20% and 60% of the overall number of neutrophils on the site of inflammation is induced into apoptosis.

The apoptosis of neutrophils may be assessed according to the method described in the examples of the invention. It may be considered that apoptosis of neutrophils is enhanced or activated when the number of neutrophils at the site of inflammation is lower when the agonist of CMKLR1 having a Resolvin E1-like capability is administered to the neutrophils, as compared to a negative control antibody. In a more particular embodiment, it may be considered that apoptosis of neutrophils is enhanced or activated when the percentage of dead neutrophils on the inflammation site is at least 20% higher, more particular at least 40% higher after 24 hours when the agonist of CMKLR1 having a Resolvin E1-like capability is administered to the neutrophils, as compared to a negative control antibody.

In a particular embodiment of the invention, the agonist of CMKLR1 having a Resolvin E1-like capability enhances the cleavage of the active form of caspase-3 in neutrophils, thereby leading to caspase-3 dependent apoptosis. The inventors indeed found that the agonists of CMKLR1 having a Resolvin E1-like capability have a positive effect on the apoptosis of neutrophils through the activation of caspase-3 (*e.g*. by enhancing the cleavage of the active form of caspase-3). It may be considered that apoptosis is enhanced or induced when the expression of caspase-3 is over 1-log, more preferably 2-log, and most preferably 3-log, after 11 hours of treatment with an agonist of CMKLR1 having a Resolvin E1-like capability as compared to a negative control. In a particular embodiment, more than 50% of neutrophils are depleted when a agonist of CMKLR1 having a Resolvin E1-like capability is in contact with neutrophils after 24 hours of treatment, as compared to control antibodies or non-humanized anti-CMKLR1 antibody of the prior art. Apoptosis of neutrophils induced by agonist of CMKLR1 having a Resolvin E1-like capability is also illustrated by the rise of the reactive oxygen species (ROS) in neutrophils treated with the antibody. Rise of ROS is known to have a role in apoptosis. Induced or enhanced apoptosis of neutrophils may be considered positive when, in a define sample, the ROS positive cells is over 10% after 5 hours of treatment with an agonist of CMKLR1 having a Resolvin E1-like capability. The method for determining the ROS positive cells may be performed by methods known in the art, like but not limited to the one described in the examples of the present invention.

The inventors also examined the effect of an agonist of CMKLR1 having a Resolvin E1-like capability on the migration and transmigration of neutrophils. Migration is the capability of neutrophils to reach the site of inflammation. Transmigration is the capability of neutrophils to migrate towards the site of inflammation through the endothelium.

Neutrophils migrate after recruitment to the inflammatory site, thereby initiating, enhancing and/or sustaining the inflammatory process, and prevent the initiation of the resolution process. The agonist of CMKLR1 having a Resolvin E1-like capability have the capability to reduce or inhibit the capability of neutrophils to migrate to the site of inflammation. The agonist of CMKLR1 having a Resolvin E1-like capability have the capability to reduce or inhibit of PMNs towards the site of inflammation, through endothelium. When neutrophils are in contact with an agonist of CMKLR1 having a Resolvin E1-like capability, their transmigration capability and/or migration capability is greatly reduced, preventing them to relocate to the site of inflammation, and exert their pro-inflammatory effects. The regulation of neutrophils migration into inflamed tissue or infected tissue may be useful to effectively treat these conditions. The inhibition of this migration capability for treating inflammatory diseases, like but not limited to chronic inflammatory diseases, is a leverage for reducing negative effects of inflammation or infection.

In a particular embodiment of the invention, the migration and transmigration of neutrophils towards the site of inflammation is prevented or reduced by the administration of anagonist of CMKLR1 having a Resolvin E1-like capability which comprises a humanized protein able to interact with Fc Receptors found on the surface of certain cells (including, among others, B lymphocytes, follicular dendritic cells, natural killer cells, macrophages, neutrophils, eosinophils, basophils, human platelets, and mast cells) in particular neutrophil or dendritic cells that contribute to the protective functions of the immune system. These receptors interact with the IgG portion of antibodies. Ig domains, like IgG, IgE, IgA and IgM. In a particular embodiment of the invention the agonist comprises at least a portion of an Ig somin, more particularly a IgG domain, and most particularly a IgG1 domain, or a humanized IgG1 domain. Therefore, in a particular embodiment, the agonist of CMKLR1 having a Resolvin E1-like capability is a humanized antibody with human constant regions derived or issued from human IgG1. In a particular embodiment, the agonist of CMKLR1 having a Resolvin E1-like capability antibody is of the human IgG1 isotype, *i.e*. the constant fragment of the heavy chain and of the light chain are derived or issued from a human IgG1 antibody constant heavy chain and light chain fragment. Accordingly, the agonist of CMKLR1 having a Resolvin E1-like capability antibody of the present invention comprises a Fc domain of IgG isotype, more particularly of human IgG1 isotype.

As illustrated in the working examples of the invention, the inventor found that when the agonist of CMKLR1 having a Resolvin E1-like capability is an antibody which comprises a Fc domain of human IgG1, it may interact with FcReceptor (FcR) expressed on the cell surface of macrophages and neutrophils along with its interaction with CMKLR1. This double interaction leads to lower the cell membrane availability (through internalization and/or shedding) of L-selectin, also known as CD62L, a cell adhesion molecule found on neutrophils, thereby reducing the migration and transmigration capability of neutrophils treated with an agonist of CMKLR1 having a Resolvin E1-like capability antibody. Indeed, the reduction of CD62L availability on the cell surface of neutrophils reduces the rolling capability of these cells on the endothelium; these cells have therefore a reduced capability to transmigrate through the endothelium. It may be considered that a neutrophil has a reduced migration and/or transmigration capability when the cell surface expression of CD62L is reduced by at least 1-log in a staining experiment, as compared to a negative control. The staining of CD62L may be performed by methods known in the art, like but not limited to the method illustrated in the examples of the invention. In a particular embodiment of the invention, the variable regions of the antibody may be associated with antibody constant regions, like IGg1, IgG2, IgG3 or IgG4 constant regions. These constant regions may be further mutated or modified, by methods known in the art, for modifying their binding capability towards Fc receptor. In a particular embodiment, the antibody or antigen-binding fragment thereof according to the invention is a humanized monoclonal antibody, in particular wherein the antibody light chain constant domain is derived from a human kappa light chain constant domain, in particular wherein the light chain constant domain comprises or consists of the sequence of SEQ ID No: 9, and wherein the antibody heavy chain constant domain is derived from a human IgG1, IgG2, IgG3, or IgG4 heavy chain constant domain, in particular wherein the antibody heavy chain constant domain comprises or consists of the amino acid sequence of SEQ ID 10, SEQ ID No. 11, SEQ ID No. 12, SEQ ID No. 13 or SEQ ID No. 14, in particular from a human IgG1 heavy chain constant domain, in particular wherein the antibody heavy chain constant domain comprises or consists of the amino acid sequence of SEQ ID No:10 or SEQ ID No. 13.

The agonist of CMKLR1 having a Resolvin E1-like capability induces internalization, or reduction, of cell surface expression of CD62L, and may therefore contribute to increase efficiency of the agonist when it is an antibody with a IgG domain. Disclosed herein is antibody compound representing a preferred embodiment, wherein in the presence of the agonist of CMKLR1 having a Resolvin E1-like capability, the cell surface expression of CD62L, and preferably of CD62L and CMKLR1, is significantly decreased. As used herein, a compound which induces the internalization and/or shedding of CD62L, and preferably of CD62L and CMKLR1 means that cells, in particular neutrophils, incubated in the presence of a compound of the invention display decreased cell surface expression of CD62L, and preferably of CD62L and CMKLR1 as compared to cells incubated in the absence of the compound of the invention. Cell surface expression is preferably measured *in vitro* after a limited incubation time and in temperature conditions as mentioned in the examples of the invention. In a preferred embodiment, the compound of the invention does induce the internalization and/or shedding of CD62L, and preferably of CD62L and CMKLR1. Thus, the cell surface expression of CD62L, and preferably of CD62L and CMKLR1 in cells incubated in the presence of the compound of the invention is reduced, or is significantly reduced, relatively to cell surface expression in cells incubated in otherwise identical conditions, but in the absence of the agonist of CMKLR1 having a Resolvin E1-like capability. In particular embodiments, when incubated at 37 °C for 30 to 45 minutes in the presence of 10 µg/mL of the agonist of CMKLR1 having a Resolvin E1-like capability, the level of CD62L, and preferably of CD62L and CMKLR1 cell surface expression is lower than 80 %, preferably lesser than 60%, and most preferably lesser than 50 %, of its level in cells incubated in the absence of the agonist of CMKLR1 having a Resolvin E1-like capability.

In a surprising manner, the inventors found that humanized IgG1 antibodies of the invention that are agonist of CMKLR1 having a Resolvin E1-like capability do not induce cytotoxicity *in vivo.* In a preferred embodiment, such antibody does not induce cytotoxicity since no decrease in CMKLR1-positive cells is observed after administration of the compound of the invention. The use of an agonist of CMKLR1 having a Resolvin E1-like capability that is a humanized IgG1 antibody may not exhibit a cytotoxic activity on CMKLR1-positive cells. The inventors illustrated that the treatment of an inflammatory condition with a IgG1 antibody of CMKLR1 having a Resolvin E1-like capability does not deplete CMKLR1+ cells, including myeloid cells, despite the known adverse effect usually associated with IgG1 derived antibody.

According to this embodiment, there is no depletion of myeloid cells, in particular neutrophils and macrophages, in patients/animals treated with IgG1 antibody agonist of CMKLR1 having a Resolvin E1-like capability humanized.

In another particular embodiment of the invention, the agonist of CMKLR1 having a Resolvin E1-like capability favors the concentration of macrophages at a site of inflammation. Macrophages are known to help the restoration of tissue function after injury, highlighting their important role in the modification of the local microenvironment of the inflammation site to restore homeostasis. The agonist compound (e.g. the antibody or antigen-binding fragment thereof, a protein, a peptide or a polypeptide) may increase the overall number of macrophages at the site of inflammation and/or modify the polarization of macrophages to favor pro-resolutive macrophages. Overall number of macrophages may be assessed by method known in the art, like but not limited to the method used in the examples of the present invention. It may be considered that the agonist of CMKLR1 having a Resolvin E1-like capability favors the concentration of macrophages at a site of inflammation when the percentage of macrophages is at least 10 % higher in a sample, like an exudate from a biological sample from a patient, treated with the compound as compared to a negative control.

In another particular embodiment of the invention, the agonist of CMKLR1 having a Resolvin E1-like capability decreases the cell surface expression of CMKLR1 and CXCR4 and/or CCR7. In a particular embodiment, the cell surface expression is considered on neutrophils and/or macrophages and/or dendritic cells. CXCR4, or chemokine receptor type 4 (CXCR-4) also known as fusin or CD184, is an alpha-chemokine receptor. CXCR4's expression is low or absent in many healthy tissues, it was demonstrated to be expressed in over 23 types of cancer, including breast cancer, ovarian cancer, melanoma, and prostate cancer. Expression of this receptor in cancer cells has been linked to metastasis to tissues. CCR7, chemokine receptor type 7, a member of the G protein-coupled receptor family is expressed in various lymphoid tissues and activates B and T lymphocytes. CCR7 has been shown to stimulate dendritic cell maturation. CCR7 is also involved in homing of T cells to various secondary lymphoid organs such as lymph nodes and the spleen as well as trafficking of T cells within the spleen. CCR7 is expressed by various cancer cells, such as nonsmall lung cancer, gastric cancer and esophageal cancer. Expression of CCR7 by cancer cells is linked with metastasis to lymph nodes.

When the agonist of CMKLR1 having a Resolvin E1-like capability binds to its target, CMKLR1, and CCR7 and/or CXCR4 are internalized. This internalization is a potent leverage for treating inflammatory related diseases, in particular in antigen presenting cells (APCs), and more particularly in dendritic cells. The internalization/decrease of these receptors leads to an efficient resolution of the inflammation. The decrease of chemokine receptors after binding of the compound of the invention to its target at the macrophage and/or dendritic cells surface limits and or prevents their migration through CXCR4 and/or CCR7 sensing.

The internalization, or the reduction of cell surface expression of one or a plurality of these receptors, induced or favored by the agonist of CMKLR1 having a Resolvin E1-like capability may contribute to increase efficiency of the agonist of the invention. Disclosed herein is an agonist representing a preferred embodiment, wherein in the presence of the agonist, the cell surface expression of CMKLR1, and CXCR4 and/or CCR7, is significantly decreased. As used herein, "agonist compound inducing the internalization of CMKLR1, and CXCR4 and/or CCR7" means that cells, in particular neutrophils and/or macrophages and/or dendritic cells, incubated in the presence of the agonist display decreased cell surface expression of CMKLR1, and CXCR4 and/or CCR7 as compared to cells incubated in the absence of the agonist of the invention. Cell surface expression is preferably measured in vitro after a limited incubation time and in temperature conditions as mentioned in the examples of the invention. In a preferred embodiment, the agonist of the invention does induce the internalization of CMKLR1 and CXCR4 and CCR7. In a preferred embodiment, the agonist of the invention reduces the cells surface expression of CMKLR1 and CXCR4 and/or CCR7. Thus, the cell surface expression of CMKLR1, andCXCR4 and/or CCR7 in cells incubated in the presence of the agonist is reduced, or is significantly reduced, relatively to cell surface expression in cells incubated in otherwise identical conditions, but in the absence of the agonist of CMKLR1 having a Resolvin E1-like capability. In particular embodiments, when incubated at 37 °C for 30 to 45 minutes in the presence of 10 µg/mL of the agonist of CMKLR1 having a Resolvin E1-like capability, the level of CMKLR1 and CXCR4 and/or CCR7 cell surface expression is lesser than 80 %, preferably lesser than 60%, and most preferably lesser than 50 %, of its level in cells incubated in the absence of the agonist of CMKLR1 having a Resolvin E1-like capability.

In a particular aspect of the invention, the inflammatory condition which is to be treated is a condition wherein the inflammation is delayed or disrupted. The inflammatory condition may be associated to a disease to be treated selected from the group consisting of inflammatory diseases, acute respiratory distress syndrome (ARDS), cancer, diabetes, immune diseases, autoimmune diseases, chronic pulmonary obstructive disease (COPD), viral pneumonia, sepsis, burn, acute coronary artery disease, rheumatoid arthritis, cystic fibrosis, infectious diseases, leukemia/lymphoma, metabolic disorders, neuroinflammation.

In a particular aspect of the invention, the inflammatory condition which is to be treated is associated with the treatment of:
- an inflammatory disease, in particular acute inflammatory diseases, chronic inflammatory diseases such as asthma, keratoconjunctivitis, periodontal disease, eczema, inflammatory bowel disease, in particular Crohn's disease or colitis, in particular ulcerative colitis or spontaneous colitis, cystic fibrosis, NASH (Nonalcoholic steatohepatitis), hepatic fibrosis, lung fibrosis, vasculitis in particular ANCA mediated vasculitis, anti-neutrophil cytoplasm antibodies-related disease (ANCA), scleroderma, in particular wherein, as a result of the administration of the treatment, the resolution of inflammation is enhanced;
- an autoimmune disease such as diabetes, in particular type I diabetes, psoriasis, lupus, rheumatoid arthritis, multiple sclerosis, Sjögren's syndrome, celiac disease, vasculitis, myasthenia gravis, or an infection disease such as sepsis, peritonitis, degenerative diseases, wound healing disorders or dry eye syndrome, in particular wherein, as a result of the administration of the treatment, the resolution of inflammation is enhanced;
- a cancer, in particular metastatic cancers, solid or liquid cancers such as carcinoma, more particularly hepatocarcinoma, in particular mammary carcinoma or colon carcinoma, or lung cancer or myeloid cancer such as leukemia, in particular a cancer wherein cancer cells express CMKLR1 or where the microenvironment of the tumor is invaded by cells expressing or overexpressing CMKLR1, in particular wherein, as a result of the administration of the treatment, the resolution of inflammation is enhanced.

In a particular aspect of the invention, the agonist of CMKLR1 having a Resolvin E1-like capability is used in the treatment of a patient suffering from NASH, hepatic fibrosis, lung fibrosis, vasculitis in particular ANCA mediated vasculitis, colitis, a colon inflammation related disease, colorectal cancer, inflammation-linked carcinogenesis, colon cancer, mesothelioma, autoimmune encephalomyelitis, psoriasis, Sjogren disease, Cutaneous inflammation, pulmonary fibrosis, neutrophil-associated diseases, sclerosis, cystic fibrosis, multiple sclerosis.

In a particular embodiment, the agonist of CMKLR1 having a Resolvin E1-like capability is associated with a further therapeutic ingredient, selected from the activators of the resolution of the inflammation. In another aspect, the invention relates to a composition comprising an anti-CMKLR1 compound as described herein, in particular a pharmaceutical composition comprising an anti-CMKLR1 compound according to the invention and a further therapeutic agent, or pharmaceutical acceptable carrier. In a particular embodiment, the invention relates to a composition comprising an anti-CMKLR1 compound according to the invention and a therapeutic agent selected from the group consisting of immunomodulatory agent, immune checkpoint blocker, and immune checkpoint activator. The term "immunotherapeutic agent," as used herein, refers to a compound, composition or treatment that indirectly or directly enhances, stimulates or increases the body's immune response against cancer cells and/or that decreases the side effects of other anticancer therapies. Immunotherapy is thus a therapy that directly or indirectly stimulates or enhances the immune system's responses to cancer cells and/or lessens the side effects that may have been caused by other anti-cancer agents. Immunotherapy is also referred to in the art as immunologic therapy, biological therapy biological response modifier therapy and biotherapy. Examples of common immunotherapeutic agents known in the art include, but are not limited to, cytokines, cancer vaccines, monoclonal antibodies and non-cytokine adjuvants. Alternatively, the immunotherapeutic treatment may consist of administering the subject with an amount of immune cells (T cells, NK, cells, dendritic cells, B cells...). Immunotherapeutic agents can be non-specific, i.e. boost the immune system generally so that the human body becomes more effective in fighting the growth and/or spread of cancer cells, or they can be specific, i.e. targeted to the cancer cells themselves immunotherapy regimens may combine the use of non-specific and specific immunotherapeutic agents. Non-specific immunotherapeutic agents are substances that stimulate or indirectly improve the immune system. Non-specific immunotherapeutic agents have been used alone as a main therapy for the treatment of cancer, as well as in addition to a main therapy, in which case the non-specific immunotherapeutic agent functions as an adjuvant to enhance the effectiveness of other therapies (e.g. cancer vaccines). Non-specific immunotherapeutic agents can also function in this latter context to reduce the side effects of other therapies, for example, bone marrow suppression induced by certain chemotherapeutic agents. Non-specific immunotherapeutic agents can act on key immune system cells and cause secondary responses, such as increased production of cytokines and immunoglobulins.

In some embodiments, the agonist of CMKLR1 having a Resolvin E1-like capability of the present invention is administered with a pharmaceutically acceptable carrier or excipient. Pharmaceutically acceptable carrier or excipient refers to a non-toxic solid, semi-solid or liquid filler, diluent, encapsulating material or formulation auxiliary of any type. The carrier can also be a solvent or dispersion medium containing, for example, water, ethanol, polyol (for example, glycerol, propylene glycol, and liquid polyethylene glycol, and the like), suitable mixtures thereof, and vegetables oils. The proper fluidity can be maintained, for example, by the use of a coating, such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminium monostearate and gelatin.

In some embodiments, the agonist of CMKLR1 having a Resolvin E1-like capability of the present invention is administered to the subject in combination with at least one further therapeutic agent, *e.g*. for treating cancers, autoimmune diseases or inflammatory diseases. Such administration may be simultaneous, separate or sequential. For simultaneous administration, the agents may be administered as one composition or as separate compositions, as appropriate. The further therapeutic agent is typically relevant for the disorder to be treated. Exemplary therapeutic agents include other anti-cancer antibodies, cytotoxic agents, chemotherapeutic agents, anti-angiogenic agents, anti-cancer immunogens, cell cycle control/apoptosis regulating agents, hormonal regulating agents, and other agents described below.

In another aspect, the invention concerns the therapeutic use of the anti-CMKLR1 agonist of the invention, especially for inducing and/or enhancing the resolution of inflammation by enhancing the apoptosis of neutrophils, in particular for inducing and/or enhancing the resolution of inflammation when said resolution is delayed or disrupted, in view of treating diseases wherein the extension over time of the inflammation is pathologic, or wherein the duration of the resolution of inflammation is pathologic.

The invention also concerns a method for promoting neutrophil apoptosis at a site of inflammation in a subject in need thereof comprising the administration to the subject a therapeutically effective amount of an agonist of CMKLR1 having a Resolvin E1-like capability. In a particular embodiment, the subject suffers from a condition associated with the inflammation. In a more particular embodiment, the subject suffers from a disease selected from, the group consisting of:
- an inflammatory disease, in particular acute inflammatory diseases, chronic inflammatory diseases such as asthma, keratoconjunctivitis, periodontal disease, eczema, inflammatory bowel disease, in particular Crohn's disease or colitis, in particular ulcerative colitis or spontaneous colitis, cystic fibrosis, NASH (Nonalcoholic steatohepatitis), hepatic fibrosis, lung fibrosis, vasculitis in particular ANCA mediated vasculitis, anti-neutrophil cytoplasm antibodies-related disease (ANCA), scleroderma, in particular wherein, as a result of the administration of the treatment, the resolution of inflammation is enhanced;
- an autoimmune disease such as diabetes, in particular type I diabetes, psoriasis, lupus, rheumatoid arthritis, multiple sclerosis, Sjögren's syndrome, celiac disease, vasculitis, myasthenia gravis, or an infection disease such as sepsis, peritonitis, degenerative diseases, wound healing disorders or dry eye syndrome, in particular wherein, as a result of the administration of the treatment, the resolution of inflammation is enhanced;
- a cancer, in particular metastatic cancers, solid or liquid cancers such as carcinoma, more particularly hepatocarcinoma, in particular mammary carcinoma or colon carcinoma, or lung cancer or myeloid cancer such as leukemia, in particular a cancer wherein cancer cells express CMKLR1 or where the microenvironment of the tumor is invaded by cells expressing or overexpressing CMKLR1, in particular wherein, as a result of the administration of the treatment, the resolution of inflammation is enhanced,
- and more particularly a disease selected from the group consisting of NASH, colitis, hepatic fibrosis, lung fibrosis, vasculitis in particular ANCA mediated vasculitis, a colon inflammation related disease, colorectal cancer, inflammation-linked carcinogenesis, colon cancer, mesothelioma, autoimmune encephalomyelitis, psoriasis, Sjogren disease, Cutaneous inflammation, pulmonary fibrosis, neutrophil-associated diseases, sclerosis, multiple sclerosis.

The invention also concerns a method for inhibiting neutrophil transmigration in a subject in need thereof comprising the administration to the subject a therapeutically effective amount of an agonist of CMKLR1 having a Resolvin E1-like capability. In a particular embodiment, the subject suffers from a condition associated with the inflammation as described herein. The invention also concerns a method for promoting macrophages proliferation at a site of inflammation in a subject in need thereof comprising the administration to the subject a therapeutically effective amount of an agonist of CMKLR1 having a Resolvin E1-like capability.

The invention also concerns a method for decreasing the cell surface expression of CMKLR1 and CCR7 and/or CXCR4 on myeloid cells, in particular macrophages and/or neutrophils and or dendritic cells, in a subject in need thereof comprising the administration to the subject a therapeutically effective amount of an agonist of CMKLR1 having a Resolvin E1-like capability.

The invention also concerns a method for promoting neutrophil apoptosis at a site of inflammation in a subject in need thereof comprising the administration to the subject a therapeutically effective amount of an agonist of CMKLR1 having a Resolvin E1-like capability. In a particular embodiment, the subject suffers from a condition associated with the inflammation. In a more particular embodiment, the subject suffers from a cancer selected from, the group consisting of primary and metastatic cancers. Examples of cancers that may treated by methods and compositions of the invention include, but are not limited to, cancer cells from the bladder, blood, bone, bone marrow, brain, breast, colon, esophagus, gastrointestinal, gum, head, kidney, liver, lung, nasopharynx, neck, ovary, prostate, skin, stomach, testis, tongue, or uterus. In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lympho epithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; non encapsulating sclerosing carcinoma; adrenal cortical carcinoma; endometroid carcinoma; skin appendage carcinoma; apocrine adenocarcinoma; sebaceous adenocarcinoma; ceruminous; adenocarcinoma; muco epidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; and roblastoma, malignant; Sertoli cell carcinoma; leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malign melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brennertumor, malignant; phyllodestumor, malignant; synovial sarcoma; mesothelioma, malignant; dysgerminoma; embryonal carcinoma; teratoma, malignant; strumaovarii, malignant; choriocarcinoma; mesonephroma, malignant; hemangio sarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblasticodontosarcoma; ameloblastoma, malignant; ameloblasticfibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythro leukemia; lymphosarcoma cell leukemia; myeloid leukemia; basophilic leukemia; eosinophilic leukemia; monocyticleukemia; mast cell leukemia; megakaryoblasticleukemia; myeloid sarcoma; and hairy cell leukemia.

The invention also concerns a method for promoting neutrophil apoptosis at a site of inflammation in a subject in need thereof comprising the administration to the subject a therapeutically effective amount of an agonist of CMKLR1 having a Resolvin E1-like capability. In a particular embodiment, the subject suffers from a condition associated with the inflammation. In a more particular embodiment, the subject suffers from an autoimmune disease selected from the group consisting of Achalasia, Addison's disease, Adult Still's disease, Agammaglobulinemia, Alopecia areata, Amyloidosis, Ankylosing spondylitis, Anti-GBM/Anti-TBM nephritis, Antiphospholipid syndrome, Autoimmune angioedema, Autoimmune dysautonomia, Autoimmune encephalomyelitis, Autoimmune hepatitis, Autoimmune inner ear disease (AIED), Autoimmune myocarditis, Autoimmune oophoritis, Autoimmune orchitis, Autoimmune pancreatitis, Autoimmune retinopathy, Autoimmune urticaria, Axonal & neuronal neuropathy (AMAN), Baló disease, Behcet's disease, Benign mucosal pemphigoid, Bullous pemphigoid, Castleman disease (CD), Celiac disease, Chagas disease, Chronic inflammatory demyelinating polyneuropathy (CIDP), Chronic recurrent multifocal osteomyelitis (CRMO), Churg-Strauss Syndrome (CSS) or Eosinophilic Granulomatosis (EGPA), Cicatricial pemphigoid, Cogan's syndrome, Cold agglutinin disease, Congenital heart block Coxsackie myocarditis, CREST syndrome, Crohn's disease, Dermatitis herpetiformis, Dermatomyositis, Devic's disease (neuromyelitis optica), Discoid lupus, Dressler's syndrome, Endometriosis, Eosinophilic esophagitis (EoE), Eosinophilic fasciitis, Erythema nodosum, Essential mixed cryoglobulinemia, Evans syndrome, Fibromyalgia, Fibrosing alveolitis, Giant cell arteritis (temporal arteritis), Giant cell myocarditis, Glomerulonephritis, Goodpasture's syndrome, Granulomatosis with Polyangiitis, Graves' disease, Guillain-Barre syndrome, Hashimoto's thyroiditis, Hemolytic anemia, Henoch-Schonlein purpura (HSP), Herpes gestationis or pemphigoid gestationis (PG), Hidradenitis Suppurativa (HS) (Acne Inversa), Hypogammalglobulinemia, IgA Nephropathy, IgG4-related sclerosing disease, Immune thrombocytopenic purpura (ITP), Inclusion body myositis (IBM), Interstitial cystitis (IC), Juvenile arthritis, Juvenile diabetes (Type 1 diabetes), Juvenile myositis (JM), Kawasaki disease, Lambert-Eaton syndrome, Leukocytoclastic vasculitis, Lichen planus, Lichen sclerosus, Ligneous conjunctivitis, Linear IgA disease (LAD), Lupus, Lyme disease chronic, Meniere's disease, Microscopic polyangiitis (MPA), Mixed connective tissue disease (MCTD), Mooren's ulcer, Mucha-Habermann disease, Multifocal Motor Neuropathy (MMN) or MMNCB, Multiple sclerosis, Myasthenia gravis, Myositis, Narcolepsy, Neonatal Lupus, Neuromyelitis optica, Neutropenia, Ocular cicatricial pemphigoid, Optic neuritisPalindromic rheumatism (PR), PANDAS, Paraneoplastic cerebellar degeneration (PCD), Paroxysmal nocturnal hemoglobinuria (PNH), Parry Romberg syndrome, Pars planitis (peripheral uveitis), Parsonage-Turner syndrome, Pemphigus, Peripheral neuropathy, Perivenous encephalomyelitis, Pernicious anemia (PA), POEMS syndrome, Polyarteritis nodosa, Polyglandular syndromes type I, II, III, Polymyalgia rheumatica, Polymyositis, Postmyocardial infarction syndrome, Postpericardiotomy syndromePrimary biliary cirrhosis, Primary sclerosing cholangitis, Progesterone dermatitis, Psoriasis, Psoriatic arthritis, Pure red cell aplasia (PRCA), Pyoderma gangrenosum, Raynaud's phenomenon, Reactive Arthritis, Reflex sympathetic dystrophy, Relapsing polychondritis, Restless legs syndrome (RLS), Retroperitoneal fibrosis, Rheumatic fever, Rheumatoid arthritis, Sarcoidosis, Schmidt syndrome, Scleritis, Scleroderma, Sjögren's syndrome, Sperm & testicular autoimmunity, Stiff person syndrome (SPS), Subacute bacterial endocarditis (SBE), Susac's syndrome, Sympathetic ophthalmia (SO), Takayasu's arteritis, Temporal arteritis/Giant cell arteritis, Thrombocytopenic purpura (TTP), Tolosa-Hunt syndrome (THS), Transverse myelitis, Type 1 diabetes, Ulcerative colitis (UC), Undifferentiated connective tissue disease (UCTD), Uveitis, Vasculitis, Vitiligo, Vogt-Koyanagi-Harada Disease.

In another embodiment, the invention, also concerns a method for *in vitro* determining if an agonist of CMKLR1 having a Resolvin E1-like capability is likely to be effective in the treatment of inflammation in a human subject. The agonist may be selected among the group consisting of an antibody or an antigen-binding fragment thereof, a peptide, a polypeptide or protein. The method comprises the steps of:
- Incubating neutrophils, in particular human neutrophils, with an effective amount of an agonist candidate;
- Quantifying the neutrophil apoptosis; in particular the apoptosis of human neutrophils,
- Selecting an agonist of CMKLR1 having a Resolvin E1-like capability candidate that enables apoptosis of neutrophils at a level of at least 20% higher as compared to a negative control.
The neutrophils may be incubated and stimulated according to the method disclosed in the examples of the invention. It may be considered that a candidate is a suitable agonist when it enhances the neutrophil apoptosis by at least 20% as compared to the neutrophil apoptosis in a control sample stimulated with a control compound (e.g. which is known to have no effect on neutrophil apoptosis). In a particular embodiment, a candidate is considered as a potent agonist when the apoptosis of neutrophils is enhanced by at least 50 % as compared to the control sample.

The method of selection may also comprise further steps. At least one further step of selection of an agonist candidate may be performed, wherein the at least one further step comprises:
- the quantification of the cell surface expression of CD62L expressed by neutrophils, in particular human neutrophils, a candidate being selected when the cell surface expression of CD62L is reduced as compared to a control;
- the quantification of the cell surface expression of CMKLR1 and CCR7 and/or CXCR4 expressed by neutrophils and/or macrophages and/or dendritic cells, a candidate being selected when the cell surface expression of CMKLR1 and CCR7 and/or CXCR4 is reduced as compared to a control.

The candidate may be considered as an agonist when the cell surface expression of CD62L expressed by neutrophils is reduced by at least 20 %, more particularly by at least 50 %, as compared to a control experiment. CD62L cell surface expression may be assessed according to the method disclosed in the examples of the present invention.

The candidate may be considered as an agonist when the cell surface expression of CMKLR1 and CCR7 and/or CXCR4 expressed by neutrophils and/or macrophages and/or dendritic cells, in particular human cells thereof, is reduced by at least 20 %, more particularly by at least 50 %, as compared to a control experiment. CMKLR1 and CCR7 and/or CXCR4 cell surface expression may be assessed according to the method disclosed in the examples of the present invention.

### FIGURE LEGENDS

**Figure 1** - **CMKLR1signaling triggering on human inflammatory macrophages** Inflammatory macrophages were generated from monocytes with 5 days of culture with M-CSF (100ng/mL) and polarized 2 days with IFNg (20ng/mL) were then cultured with coated Isotype (black cross) or 2G1 (square) for different times. Whole proteins of each condition were extracted and an ELISA of pErk or pAkt was performed on those lysates.
**Figure 2** - **CMKLR1-positive myeloid cells in a LPS model**
   LPS was injected intraperitoneally (5mg/kg) and cells from the peritoneum and the spleen were collected after 24h for flow cytometry analysis of ChemR23 expression.
**Figure 3** - **Neutrophil apoptosis. A) Survival/Mortality of neutrophils B) Caspase-3 expression C) ROS production**
   Neutrophils were isolated from blood of healthy volunteers and cultured for 24h (Mortality analysis), or 4h, 6h, 11h (Caspase-3 assay), or 5h (ROS assay) on coated Iso ctrl (cross), chimeric anti-ChemR23 2G1 (square) or different humanized versions of the 2G1 (diamonds). Mortality of PMN was analyzed by incubating PMN with specific markers of the mortality and viability and then counting by picture analysis. Caspase-3 is revealed by western blot and intensity of signals was determined with a software. ROS production was revealed with a specific marker and analyzed on pictures.

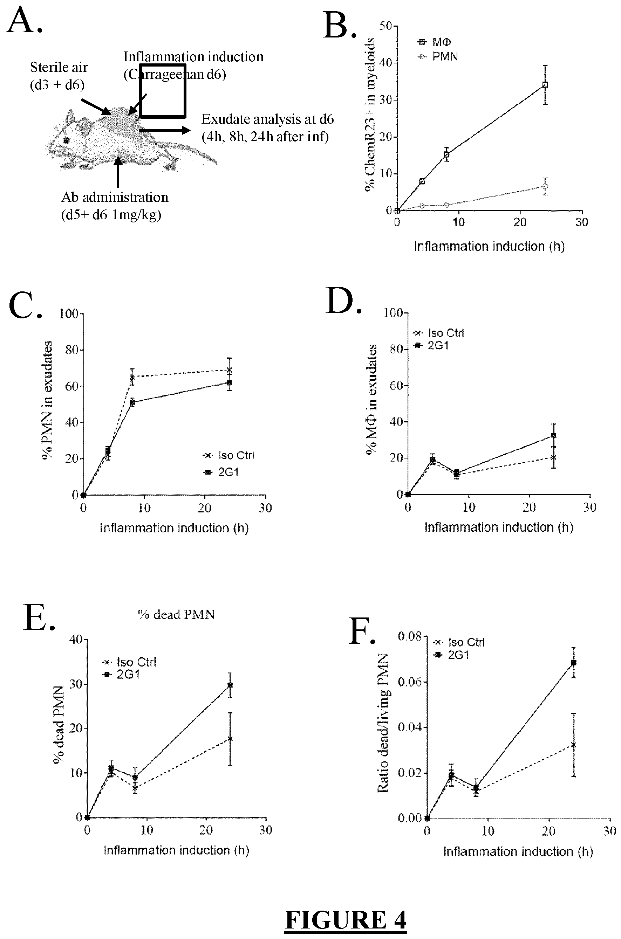

**Figure 4** **- in vivo neutrophils apoptosis** - **A) Experimental proceedings** - **B) ChemR23 expression C) neutrophils frequency in exudates D) macrophages frequency in exudates G) neutrophils mortality F) Ratio dead/living neutrophils** Sterile air was injected two times at d3 and d6 and inflammation was initiated with the injection of carrageenan. Exudates were collected at different times and stained for flow cytometry analysis.

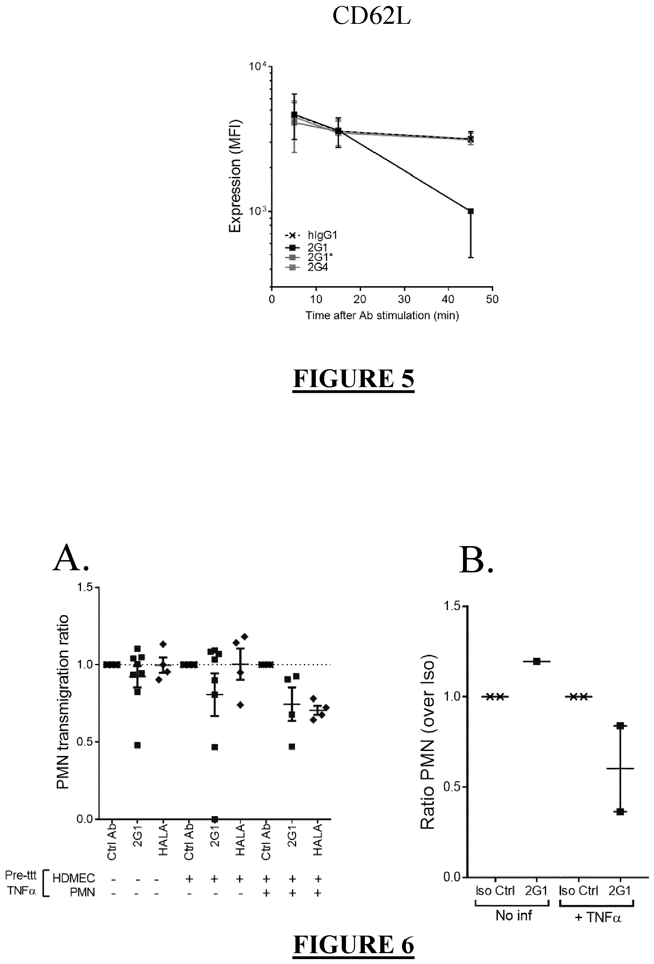

**Figure 5** **- CD62L expression on neutrophils**
   PMN were incubated for different times on coated Ab and then stained for CD62L expression analyzed by flow cytometry.
**Figure 6** **- Neutrophils transmigration ratio- A) transmigration ratio of PMNs in healthy patients** - **B) Ratio PMNs in inflamed patients suffering from ANCA** Endothelial cells were coated and +/- activated with 100U/mL of TNFa overnight. Then PMN were added with or without TNFa at 100U/mL and Ab for two hours. The transmigrated PMN were collected and analyzed by flow cytometry.

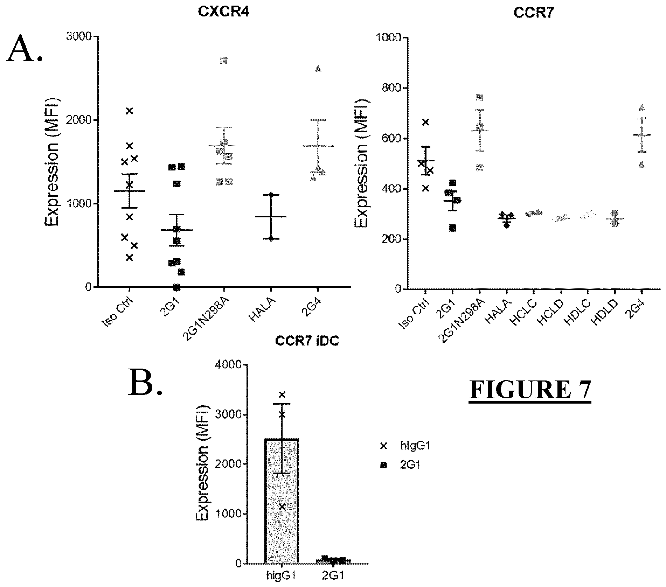

**Figure 7** **- Cell surface expression of CXCR4 and CRC7** - **A) Expression in inflammatory macrophages of CXCR4 and CCR7 incubated with different antibodies** - **B) Expression of CCR7 in dendritic cells**
   Inflammatory macrophages were generated from monocytes with 5 days of culture with M-CSF (100ng/mL) and polarized 2 days with IFNg (20ng/mL) and then cultured with coated Ab. Dendritic cells were induced with GM-CSF (50ng/mL) and IL-4 (20ng/mL) for 6 days and cultured with coated Ab during the differentiation. CXCR4 and CCR7 were revealed by flow cytometry.

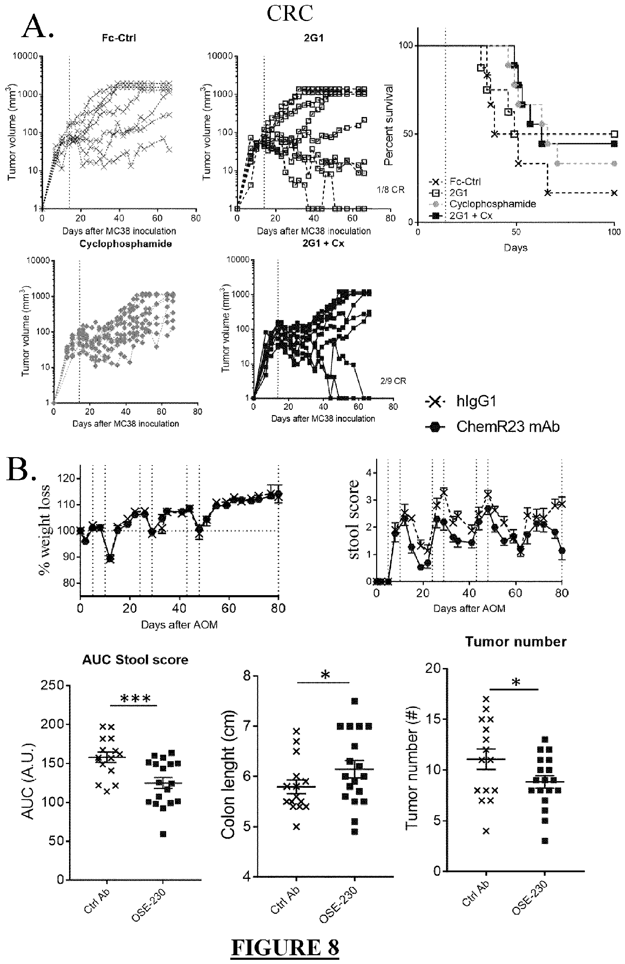

**Figure 8** **- CRC model A) Tumor cell inoculated model of CRC** : **tumor development and survival B) Chemically and inflammation induced CRC** Tumor inoculation was performed with 0.5M of MC38 CRC cell line subcutaneously. Mice were injected three times a week with 1mg/kg of 2G1 or Ctrl Ab for 3 weeks starting at d4 after tumor induction. Cyclophosphamide was injected I.P. once at 100mg/kg. Tumor development was assessed three times a week and survival curves were established when mice developed a tumor > 1000mm3.

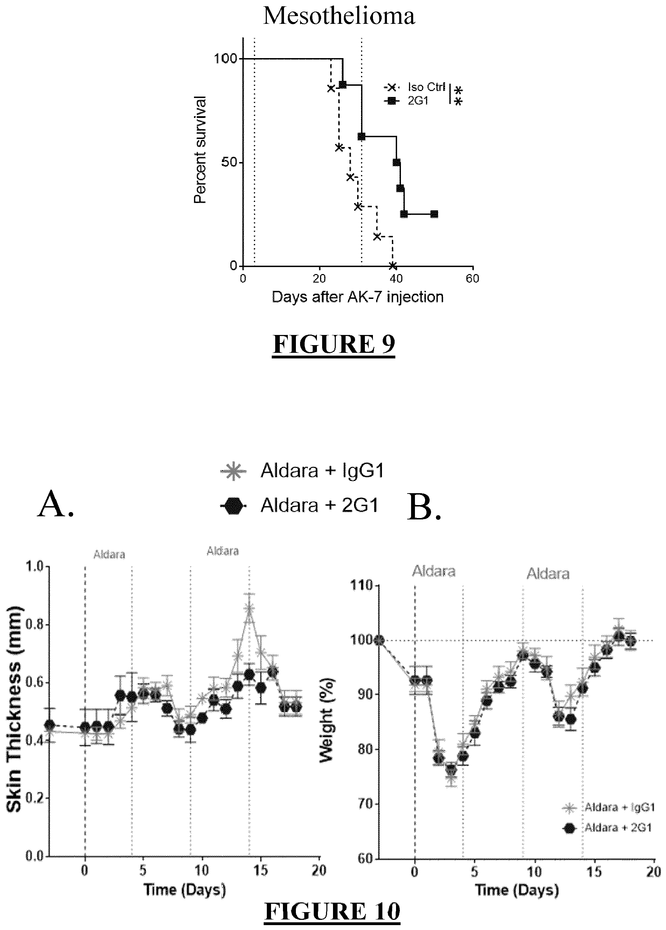

**Figure 9** **- Mesothelioma model** - **Survival**
   AK-7 cells (3M) were injected in the pleural cavity and mice received either Iso ctrl or 2G1 three times a week for 3 weeks starting at d4 at 1mg/kg.
**Figure 10** **- Effect of CMKLR1 agonist (2G1) on an autoimmune disease such as mice psoriasis model..** Aldara treated mice for 4 to 6 consecutive days were injected intra-peritoneally with: 2G1 (●) or isotype control antibody (x). **A.** thicknesses and **B** weight.

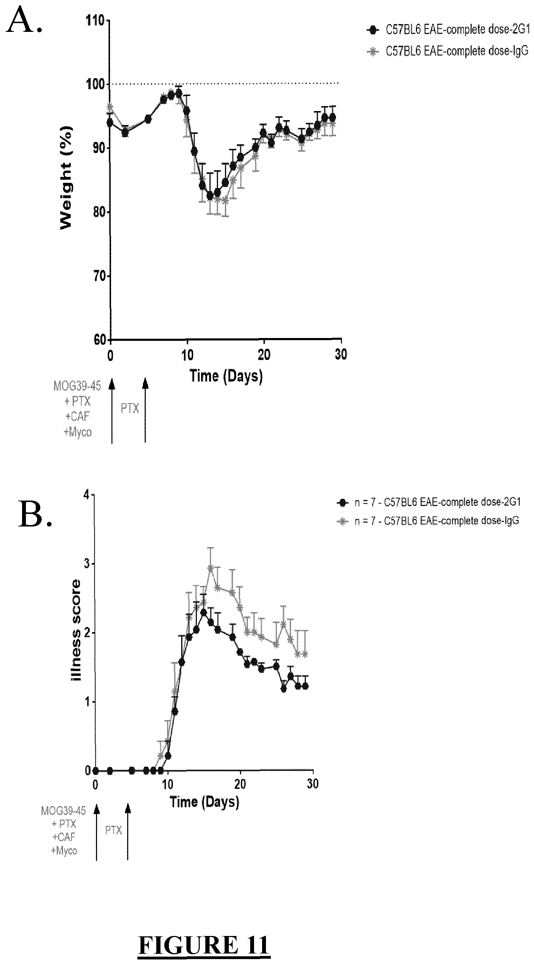

**Figure 11** **- autoimmune encephalomyelitis experimental model. - A) Weight variation over time B) illness score**
   Lesions in the central nervous system were induced by injection of the immunogenic MOG peptide combined with adjuvants. Treatment (2G1) or Isotype control were administered at 1mg/kg when animals had a clinical score equal to 2 meaning the central nervous system is already affected by T cell activity, until the end of the experiment.

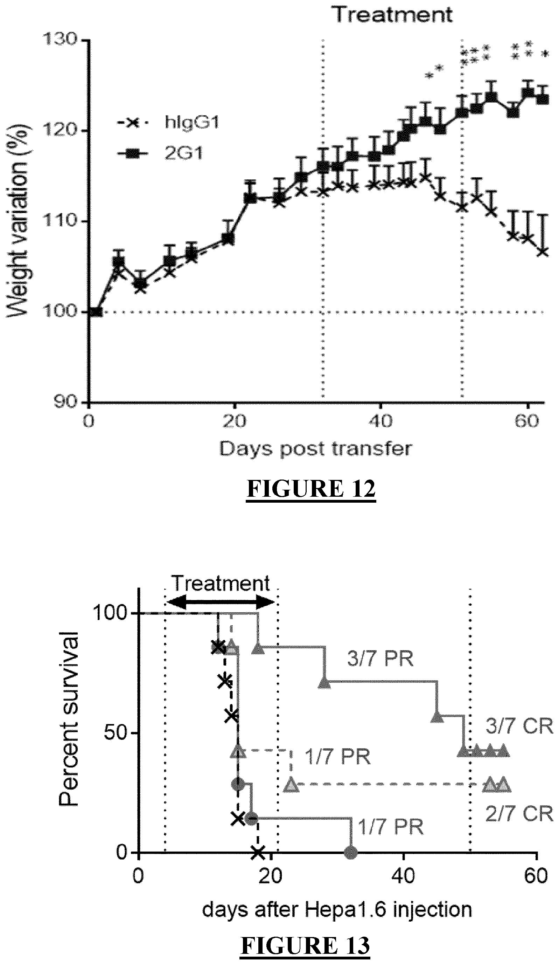

**Figure 12****. Effect of an anti-CMKLR1 antibody in the CD45Rb^{high} T-cell transfer chronic colitis mouse model.**
   Weight variation of treated animals was followed up to sixty days. Animals were treated with isotype control hlgG1 (x) or anti-CMKLR1 antibody (■).
**Figure 13** **- Effect of an anti-CMKLR1 antibody on an Hepatocarcinoma mice model (HCC model).** Anti-tumor effect of anti-CMKLR1 antibody (2G1, 0.8mg/kg) i.p. administration three times a week for 2 weeks in combination (▲) or not (●) with two injections on day 4 and 8 of anti-PD1 mAb (*RMP1-14* clone, 8mg/kg) or with injections (twice a week) of anti-PD-1 antibody alone (Δ). Mice have been treated during 2 weeks in an orthotropic model of murine hepatoma (2.5.10^6 of Hepa 1.6 cells injected through the portal vein on day 0). Isotype control antibody was used at 0,8 mg/kg three times a week for 2 weeks. Mice response were considered partial (PR) when mice survive few days to 1 month after stop of treatment or complete (CR) when mice survive over 1 month or cured when they survive three times longer than the time necessary to all control mice dye.

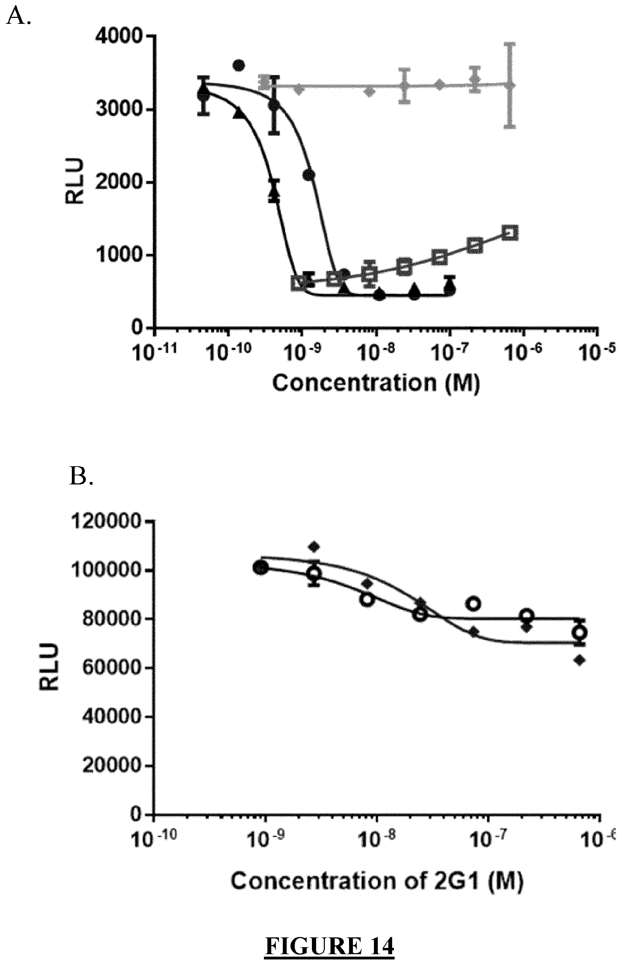

**Figure 14****. Competition study on chemerin-CMLKR1 interaction with anti-CMKLR1 antibodies. A.** Inhibition of the cAMP by Chemerin from two different providers were tested from DiscoverX (•) or R&D System (A) or combined with anti-CMKLR1 antibody (2G1) alone (□) or combined with chemerin from DiscoverX (□). **B.** beta Arrestin activation in presence of anti-CMKLR1 antibody at different concentrations from 1µM to 1nM and Chemerin 2nM (□) or 6nM (•).

### EXAMPLES

In the following examples, antibody 2G1 comprises the heavy chain variable domain of SEQ ID No. 5, and the light chain variable domain of SEQ ID No. 6; antibody HALA comprises the heavy chain variable domain of SEQ ID No. 7, and the light chain variable domain of SEQ ID No. 8. Other antibodies comprising HB, HC or HD heavy chain variable domain and VL VC or VD light variable domains are derived from HA the heavy chain variable domain HA. And the light chain variable domain LA, respectively.

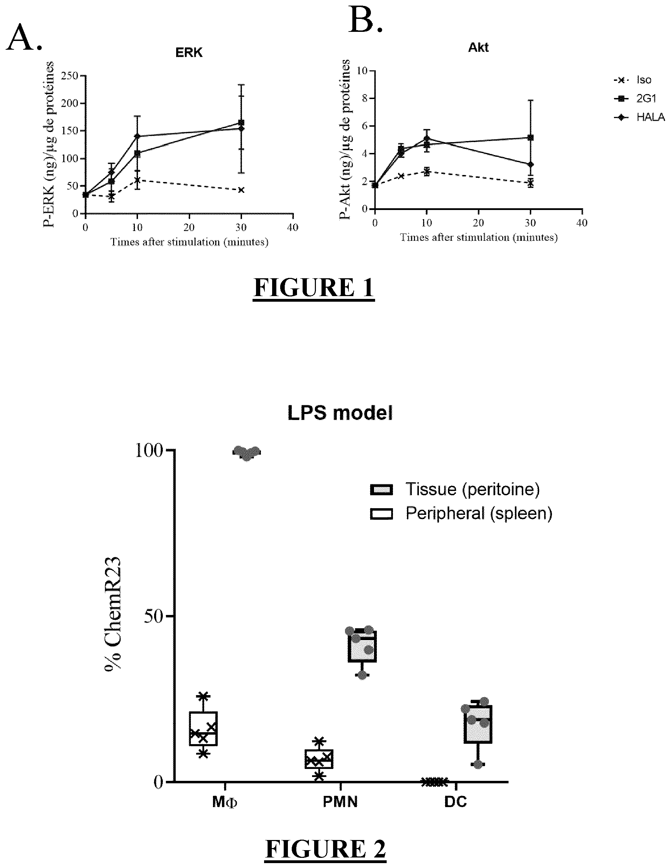

### Example 1. CMKLR1 signaling induced by the agonist of the invention (Figure 1)

As illustrated in Fig. 1A, the use of an angonist of CMKLR1 (2G1 and HALA) induces the phosphorylation of ERK and Akt when cells are stimulated with an agonist of CMKLR1 (**Fig. 1B**).

### Example 2. Effect of the use of an agonist of CMKLR1 in a LPS inflammation model (Figure 2)

MATERIAL AND METHODS. 8-weeks old male C57BI/6J mice were injected intraperitoneally with LPS at 5 mg/kg. Peritoneal fluid & spleen were harvested 24h later and stained for ChemR23 expression by flow cytometry.

RRSULTS. A few proportion of splenic macrophages (F4/80+), PMN (CD11b+/Ly6G+) and Dendritic cells (CD11c+) expressed ChemR23, 10%, 5% and 0% respectively (white squares). The proportion is much more elevated at the inflammation site where 100% of macrophages, 40% of PMN and 10% of DCs expressed CMKLR1 (black squares). This demonstrates the impact of LPS inflammation on the CMKLR1 resolution receptor expression by myeloid cells, thereby indicating that an agonist of this receptor interacts with cells implicated in the inflammation process.

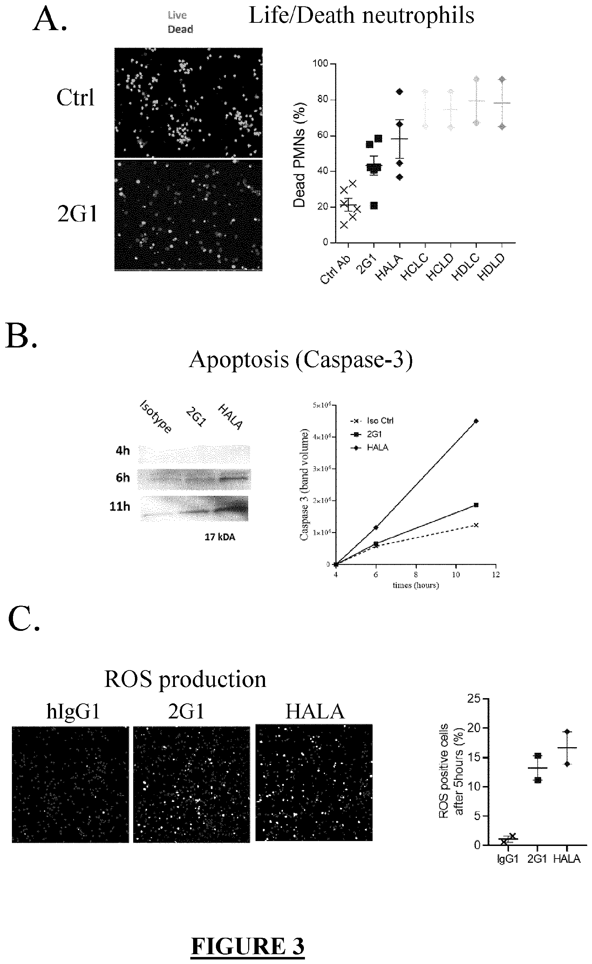

### Example 3. Neutrophils apoptosis and mortality induced by a CMKLR1 agonist (Figure 3 and Figure 4)

MATERIAL AND METHODS. PMNs from healthy volunteers were incubated in culture medium with coated Ab at 10µg/mL for different times and collected for Caspase-3 staining analyzed by Western Blot. The intensity of Capase-3 expression was calculated on WB. For the ROS test, PMNs from healthy volunteers were incubated in culture medium with coated Ab at 10µg/mL for 24h or 5h and stained either with a dead/viability kit or a specific marker of reactive oxygen species (ROS) respectively.

RESULTS. As illustrated on **Fig. 3A****,** neutrophils life/death ratio is higher in cells treated with an antibody of the invention, thereby illustrating the effect on these cells of the agonist of the invention. As illustrated on **Fig. 3B****,** the administration of an anti-CMKLR1 agonist enhances caspase-3 activity, as compared to cells treated with a control antibody. The antibody HALA exhibits higher effect on caspase 3 activity as compared to the 2G1 antibody. 2G1 WT and HALA increases caspase-3 cleavage, which means that ChemR23 triggering leads to Caspase-3-dependent apoptosis. On **Fig. 3C****,** it can be seen that the percentages of positive cells obtained by analyzing the pictures with ImageJ software is higher when cells are treated with an agonist of CMKLR1. 2G1 and all humanized variants of 2G1 increase the mortality of PMN after 24 hours. 2G1 and HALA variants increase the ROS production by PMN after 5h.

In another model, which is illustrated on **Fig. 4A****,** confirmative data on neutrophil apoptosis have been obtained. As illustrated on **Fig. 4B****,** the percentage of ChemR23-positive cells (macrophages and neutrophils) is increased when inflammation is induced. But, the percentage of neutrophils in exudates is not significantly decreased in animals treated with the anti-CMKLR1 antibody (**Fig. 4C**; black square), while the overall percentage of macrophages in exudates is slightly enhanced (**Fig. 4D**). This indicates that the administration of the CMKLR1 agonist does not reduce the overall number of myeloid cells in the exudates, and has an effect on apoptosis of the neutrophils mainly at the site of inflammation. As illustrated on **Fig. 4E** and **Fig. 4F****,** the percentage of dead neutrophils is increased when the agonist of CMKLR1 is administered, as well as their death. These results illustrate the positive effect of the agonist of CMKLR1 for treating a delay in the resolution of the inflammation, since the neutrophil population is impacted not in exudates but at the site of inflammation.

### Example 4. CD62L expression (Figure 5)

MATERIAL AND METHODS. PMN from healthy volunteers were incubated in culture medium with coated Ab at 10µg/mL for different times and collected for CD62L staining analyzed by flow cytometry.

RESULTS. 2G1, only on the IgG1 format, leads to the decrease of CD62L on the PMN surface over the time as illustrated on **Fig. 5****.**

### Example 5. Neutrophils transmigration capability (Figure 6)

MATERIAL AND METHODS. Human endothelial cells (HDMEC) were incubated for 24h in the transwell coated with gelatin and activated overnight with TNF-alpha at 100U/mL or without inflammatory conditions. Then, PMN from healthy volunteers or ANCA patients were incubated in transwell containing the monolayer of HDMEC for 4 hours. Antibodies (Iso Ctrl and 2G1) were added at 10µg/mL +/- TNF-alpha at 100U/mL during the 4hours of the transmigration assay. The lower transmigrated part of the transwell was collected and the transmigrated PMN were counted by flow cytometry using counting beads.
RESULTS. Neutrophils treated with a CMKLR1 agonist have a lower transmigration capability as compared to cells treated with control compounds (**Fig. 6A**). 2G1 avoids PMN transmigration through endothelial monolayer especially under inflammatory condition when PMN and endothelial cells were activated with TNFa in healthy volonters and in AIDs' patients (**Fig. 6B**).

### Example 6. CCR7 / CXCR4 cell surface expression (Figure 7)

MATERIAL AND METHODS. Macrophages were generated from monocytes of healthy volunteers with 100ng/mL of M-CSF for 5 days. Then, macrophages were collected and incubated with coated mAb at 10mg/mL in presence of 20ng/mL of IFNg in order to obtain M1 inflammatory macrophages. Then, M1 were phenotyped for CXCR4 and CCR7 by flow cytometry and cytokines released in the supernatant were dosed by ELISA. Dendritic cells were generated from monocytes of healthy volunteers with 50ng/mL of GM-CSF and 20ng/mL of IL-4 for 6 days. Then, DC were phenotyped for CCR7 by flow cytometry.
RESULTS. On **Fig. 7A****,** it is clear that CMKLR1 agonist (2G1 and HALA) induces a reduction in the expression of CXCR4 and CCR7 (**Fig. 7A**). When ChemR23 is targeted with a specific IgG1 format, the expression of chemokine receptors CXCR4 and CCR7, which are known to heterodimerize with ChemR23, is reduced on macrophages and Dentritic Cells which are efficient antigen presenting cells (**Fig. 7B**). This means that 2G1 and humanized variants therefore prevent migration of inflammatory APC from inflammatory site to secondary lymphoid organs.

### Example 7. Effect of CMKLR1 agonist on CRC model (Figure 8)

As illustrated on **Fig. 8A****,** the tumor volume is reduced in animals treated with the anti-CMKLR1 antibody of the invention, as compared to the control antibody. In several cases, complete remission is also observed, thereby illustrating the positive effect of a compound according to the invention for treating CRC. As shown, the percent survival is also enhanced in animals treated witht an agonist of CMKLR1.
Moreover, as exemplified on **Fig. 8B****,** the treatment with an anti-CMKLR1 monoclonal antibody (OSE-230) leads to a reduction of the stool score, and the reduction of tumor number.

### Example 8. Effect of CMKLR1 agonist on mesothelioma model (Figure 9)

Mice treated according to the method explained in the description of the figure with an agonist of CMKLR1 have a higher survival rate than mice treated with a control antibody, thereby illustrating the positive effect of a compound according to the invention for treating mesothelioma (**Fig. 9**).

### Example 9. Effect of CMKLR1 agonist on Imiquimod-induced psoriasis-like skin inflammation model (Figure 10)

Aldara® cream which is known to induce psoriasis in mice was used on male C57BI/6 mice (8-10-week old). Mice received a daily topical dose of Aldara. Treatments (anti-CMKLR1 agonist or control compound) were injected intra-peritoneally:
RESULTS: Anti-CMKLR1 agonist (2G1) reduces the thickness of the skin (**Fig. 10A**) after Aldara administration (see for example on day 15), while the weight of the animals is not impacted by the administration of the agonist (**Fig. 10B**).These results suggest an application for agonist anti-CMKLR1 compound therapy on psoriasis mice model, illustrative of an autoimmune disease.

### Example 10. Effect of CMKLR1 agonist on autoimmune encephalomyelitis experimental model (Figure 11)

In this model, the curative administration of the antibody of the invention in EAE model does not lead to the reduction of the weight (**Fig. 11A**), as compared to animals treated with a control antibody. But the illness score is significantly reduced when the treatment is performed with the anti-CMKLR1 antibody (**Fig. 11B**). The anti-CMKLR1 antibody leads to a great improvement of the illness score as soon as ten-day post treatment as compared to the control, since the score is approximately 30 % lower in animals treated with the agonist compound. It is therefore illustrated that a treatment with an anti-CMKLR1 compound is effective for treating autoimmune encephalomyelitis.

### Example 11. CD45Rb^{high} T-cell transfer chronic colitis mouse model (Figure 12)

METHOD. CD45Rb^{high} CD4 T cells were isolated from the spleen of naive mice and sorted on an ARIA FACS after a negative selection of the CD4 T cells by magnetic sorting, then injected intraperitoneally at 0,5.10⁶ cells in 100µL of PBS into 6-weeks old female Rag1 knock-out mice. Anti-CMKLR1 antibody (2G1) or an isotype control were administered from day 32 after the CD45Rb^{high} CD4 T cell transfer for 3 weeks three times a week at 1mg/kg. The follow-up of weight was evaluated three times a week and the weight variation was determined over the initial weight. * p < 0.05, ** p < 0.01.
RESULTS. **Fig. 12** presents the percentage of weight variation over the time post antibody administration, of animals treated with anti-CMLKR1 antibody or an isotype control. Both groups presented the same initial weight evolution over the first 30 days after treatment and started to differentiate at day 35. Mice treated with the anti-CMKLR1 continue to gain weight while, in contrast, control mice start to lose weight indicating development of chronic colitis as anticipated in this control group. The inventors confirm in a third model of colitis, here in a chronic model of inflammation, that anti-CMKLR1 antibodies of the invention could be of interest to treat chronic inflammatory and autoimmune diseases such as colitis.

### Example 12. Anti-tumor effect on the overall survival of mice hepatocarcinoma tumor model (Figure 13)

METHOD. Mice were anesthetized with a cocktail of xylazine/ketamine. After a laparotomy, tumoral Hepa 1.6 cells were injected in PBS through the portal vein (2,5.10⁶ cells/100 µL) in PBS. The treatment was started 4 days after tumor injection. The anti-CMKLR1 antibody (2G1clone) and the hlgG1 istotype control were injected at 0.8mg/kg three times per week during 2 weeks. The anti-PD1 monoclonal antibody was injected twice a week during 2 weeks intraperitoneally in PBS (8mg/kg). Combination anti-CMKLR1 and anti-PD1 antibodies was tested as well (0.8 mg/kg and 8mg/kg respectively). The Overall survival was followed during sixty days and the percentage of survival in each condition was reported Figure 22.

RESULTS. As shown in **Figure 13****,** animals treated with the anti-CMKLR1 or the anti-PD1 antibodies had seen their survival rate prolonged only for 1 animal on 7 treated (15% of treated animals) indicating a partial response (PR). However, animals treated with a combination of anti-PD1 and anti-CMKLR1 antibodies allows a significant increase of the survival rate (from 15% to 45%) with animals alive 60 days after treatment, indicating a complete response (CR). This result indicates an unexpected efficiency of the therapeutic combination (anti-PD1/antiCMKLR1 antibodies) on HCC tumor model.

### Example 13. Effect of an Agonist of CMKLR1 on the beta-arrestin pathay (Figure 14)

The day before the experiment CHO-K1 CMKLR1 Gi cells (Discover'X ref 95-0080C2) were plated in pre-warmed cell reagent then plated in a 96-well plate at 100 µl/well of cells as (Discover'X ref 15-103) and incubated 24 hours at 37°C, in a 5% CO2 humidified incubator for 24 hours.
A mix of Chemerin agonist (6x in 7-point series of 3-fold dilutions from 10⁻⁷µM to 10⁻¹⁰M) (Discover'x ref 92-1036 or 2324-CM-025 from R&D Systems) and forskolin (40µM) (a cAMP activator) (Discover'x ref 92-0005) was added to the cells during 30 min at 37°C; or cells were pre-incubated 30 min at 37°C with anti-CMKLR1 antibody (serial dilution : 6X in 7-point series of 3-fold dilutions from 1µM to 1nM). Then a mix of chemerin (2nM) + forskolin (60nM) was added to the cells 30 min at 37°C. For the detection cAMP, antibody reagent and cAMP working detection solution was added to the plate for 1 hour at room temperature, then cAMP solution A was added, and cells incubated 3 hours at room temperature in the dark. Bioluminescence was read with a plate reader with 0,5s integration.

RESULTS. In order to test if the antibody of the invention is an antagonist of the Chemerin-induced CMKLR1 activation, two assays were performed, and the results are presented on **Figure 14****.** Chemerin-induced inhibition of forskolin-dependent cAMP production is shown on Figure **14A** **(black circles or white squares);** anti-CMLKR1 antibody of the invention could not revert this inhibition of production (**black circles or white squares**) as compared to the control (grey diamonds). Chemerin-induced activation of the beta-arrestin presented Figure **14B****,** show that the anti-CMKLR1 antibody of the invention did not significantly modify chemerin-dependent activation of the beta-arrestin (white circles as compared to black diamonds). The antibodies of the invention do not have an antagonist activityof the CMLKR1-Chemerin interaction. Furthermore, the antibodies of the invention are not able to induce chemerin-induced CMKLR1 signaling pathway, confirming that these antibodies are not agonist of chemerin of the CMLKR1 pathway.

## Claims

1. An agonist of anti-Chemerin Like Receptor 1 (CMKLR1) having a Resolvin E1-like capability , for use in the treatment of a patient suffering from an inflammatory condition, in particular an inflammatory condition wherein the resolution phase of the inflammation is delayed or disrupted, wherein the agonist of CMKLR1 having a Resolvin E1-like capability is selected from the group consisting of an antibody or antigen-binding fragment thereof, a peptide, a polypeptide and a protein, and wherein the agonist: :
- induces or activates the apoptosis of neutrophils at the site of inflammation,
- and/or inhibits or decreases the transmigration capability of neutrophils through endothelium towards the site of inflammation,
- and/or inhibits or decreases the migration of macrophages and/or of dendritic cells from the site of inflammation to secondary lymphoid organs and/or towards the site of inflammation.

2. The agonist according to claim 1 for use according to claim 1 which binds to the third extra cellular loop of CMKLR1.

3. The agonist according to claim 1 or 2, for use according to claim 1 or 2, wherein the agonist is an antibody, in particular a humanized antibody, or antigen-binding fragment thereof.

4. The agonist according to any one of claims 1 to 3, for the use of any one of claims 1 to 3, wherein the inflammatory condition is related to a cancer, an autoimmune disease, an infection, a chronic inflammatory disease, a chronic infection or sepsis, diagnosed in the patient.

5. The agonist according to any one of claims 1 to 4, for use according to any one of claims 1 to 4, wherein the agonist increases the concentration of pro-resolutive inflammation macrophages at the site of inflammation.

6. The agonist according to any one of claims 1 to 5, for use according to any one of claims 1 to 5, wherein the agonist antibody induces the internalization of CMKLR1 and CCR7 and/or CXCR4, in particular of CMKLR1 and CCR7 and CXCR4, expressed by myeloid cells, in particular by macrophages and/or dendritic cells.

7. The agonist according to any one of claims 1 to 6, for use according to any one of claims 1 to 6, wherein the agonist antibody reduces the cells surface expression of CMKLR1 and CCR7 and/or CXCR4, in particular of CMKLR1 and CCR7 and CXCR4, expressed by myeloid cells, in particular by macrophages and/or dendritic cells.

8. The agonist according to any one of claims 1 to 7, for use according to any one of claims 1 to 7, wherein the agonist induces the internalization and/or shedding of CD62L expressed by myeloid cells, in particular by neutrophils.

9. The agonist according to any one of claims 1 to 8, for use according to any one of claims 1 to 8, wherein the agonist reduces the cell surface expression of CD62L expressed by myeloid cells, in particular by neutrophils.

10. The agonist according to any one of claims 1 to 9, for use according to any one of claims 1 to 9, wherein the agonist is a humanized antibody comprising a domain suitable for interaction with a Fc receptor, particularly a IgG domain, and more particularly a IgG1 constant fragment or a constant fragment issued or derived from a human IgG1.

11. The agonist according to any one of claims 1 to 10, for use according to any one of claims 1 to 10, which does not activate the β-arrestin signaling pathway in CMKLR1-positive cells *in vitro* and/or *in vivo.*

12. The agonist according to any one of claims 1 to 11, for use according to any one of claims 1 to 11, wherein the administration of the agonist induces or sustains the resolution stage of the inflammation.

13. The agonist according to any one of claims 1 to 12, for use according to any one of claims 1 to 12, wherein the inflammatory condition is selected from the group consisting of:
- an inflammatory disease or a chronic inflammatory disease, in particular acute inflammatory diseases, such as asthma, keratoconjunctivitis, periodontal disease, eczema, inflammatory bowel disease, in particular Crohn's disease or colitis, in particular ulcerative colitis or spontaneous colitis, cystic fibrosis, NASH (Nonalcoholic steatohepatitis), hepatic fibrosis, lung fibrosis, vasculitis in particular ANCA mediated vasculitis, anti-neutrophil cytoplasm antibodies-related disease (ANCA), scleroderma, in particular wherein, as a result of the administration of the treatment, the resolution of inflammation is enhanced;
- an autoimmune disease such as diabetes, in particular type I diabetes, psoriasis, lupus, rheumatoid arthritis, multiple sclerosis, Sjögren's syndrome, celiac disease, vasculitis, myasthenia gravis, or an infection disease such as sepsis, peritonitis, degenerative diseases, wound healing disorders or dry eye syndrome, in particular wherein, as a result of the administration of the treatment, the resolution of inflammation is enhanced;
- a cancer, in particular metastatic cancers, solid or liquid cancers such as carcinoma, more particularly hepatocarcinoma, in particular mammary carcinoma or colon carcinoma, or lung cancer or myeloid cancer such as leukemia, in particular a cancer wherein cancer cells express CMKLR1 or where the microenvironment of the tumor is invaded by cells expressing or overexpressing CMKLR1, in particular wherein, as a result of the administration of the treatment, the resolution of inflammation is enhanced,
- and more particularly a disease selected from the group consisting of NASH, colitis, hepatic fibrosis, lung fibrosis, vasculitis in particular ANCA mediated vasculitis, a colon inflammation related disease, colorectal cancer, inflammation-linked carcinogenesis, colon cancer, mesothelioma, autoimmune encephalomyelitis, psoriasis, Sjogren disease, Cutaneous inflammation, pulmonary fibrosis, neutrophil-associated diseases, sclerosis, multiple sclerosis.

14. A method for *in vitro* determining if an agonist of CMKLR1 having a Resolvin E1-like capability is likely to be effective in the treatment of inflammation in a human subject, the method comprising the steps of:
- Incubating neutrophils with an effective amount of an agonist candidate;
- Quantifying the neutrophil apoptosis; in particular the apoptosis of human neutrophils,
- Selecting an agonist of CMKLR1 having a Resolvin E1-like capability candidate that enables apoptosis of neutrophils at a level of at least 20% higher as compared to a negative control.

15. The method according to claim 14, wherein at least one further step of selection of an agonist candidate is performed, wherein the at least one further step comprises:
- the quantification of the cell surface expression of CD62L expressed by neutrophils, a candidate being selected when the cell surface expression of CD62L is reduced as compared to a control;
- the quantification of the cell surface expression of CMKLR1 and CCR7 and/or CXCR4 expressed by neutrophils and/or macrophages and/or dendritic cells, a candidate being selected when the cell surface expression of CMKLR1 and CCR7 and/or CXCR4 is reduced as compared to a control.


SEQUENCE LISTING

<110>  OSE IMMUNOTHERAPEUTICS

<120>  CMKLR1 AGONISTS HAVING A RESOLVIN E1-LIKE CAPABILITY AND THEIR
THERAPEUTIC APPLICATIONS

<130>  B14129/AD/HLQ

<140>  EPxxxxxxxx.x
<141>  2019-10-04

<160>  14

<170>  PatentIn version 3.5

<210>  1
<211>  373
<212>  PRT
<213>  artificial sequence

<220>
<223>  Synthetic sequence

<400>  1

Met Arg Met Glu Asp Glu Asp Tyr Asn Thr Ser Ile Ser Tyr Gly Asp
1               5                   10                  15

Glu Tyr Pro Asp Tyr Leu Asp Ser Ile Val Val Leu Glu Asp Leu Ser
            20                  25                  30

Pro Leu Glu Ala Arg Val Thr Arg Ile Phe Leu Val Val Val Tyr Ser
        35                  40                  45

Ile Val Cys Phe Leu Gly Ile Leu Gly Asn Gly Leu Val Ile Ile Ile
    50                  55                  60

Ala Thr Phe Lys Met Lys Lys Thr Val Asn Met Val Trp Phe Leu Asn
65                  70                  75                  80

Leu Ala Val Ala Asp Phe Leu Phe Asn Val Phe Leu Pro Ile His Ile
                85                  90                  95

Thr Tyr Ala Ala Met Asp Tyr His Trp Val Phe Gly Thr Ala Met Cys
            100                 105                 110

Lys Ile Ser Asn Phe Leu Leu Ile His Asn Met Phe Thr Ser Val Phe
        115                 120                 125

Leu Leu Thr Ile Ile Ser Ser Asp Arg Cys Ile Ser Val Leu Leu Pro
    130                 135                 140

Val Trp Ser Gln Asn His Arg Ser Val Arg Leu Ala Tyr Met Ala Cys

145                 150                 155                 160

Met Val Ile Trp Val Leu Ala Phe Phe Leu Ser Ser Pro Ser Leu Val
                165                 170                 175

Phe Arg Asp Thr Ala Asn Leu His Gly Lys Ile Ser Cys Phe Asn Asn
            180                 185                 190

Phe Ser Leu Ser Thr Pro Gly Ser Ser Ser Trp Pro Thr His Ser Gln
        195                 200                 205

Met Asp Pro Val Gly Tyr Ser Arg His Met Val Val Thr Val Thr Arg
    210                 215                 220

Phe Leu Cys Gly Phe Leu Val Pro Val Leu Ile Ile Thr Ala Cys Tyr
225                 230                 235                 240

Leu Thr Ile Val Cys Lys Leu Gln Arg Asn Arg Leu Ala Lys Thr Lys
                245                 250                 255

Lys Pro Phe Lys Ile Ile Val Thr Ile Ile Ile Thr Phe Phe Leu Cys
            260                 265                 270

Trp Cys Pro Tyr His Thr Leu Asn Leu Leu Glu Leu His His Thr Ala
        275                 280                 285

Met Pro Gly Ser Val Phe Ser Leu Gly Leu Pro Leu Ala Thr Ala Leu
    290                 295                 300

Ala Ile Ala Asn Ser Cys Met Asn Pro Ile Leu Tyr Val Phe Met Gly
305                 310                 315                 320

Gln Asp Phe Lys Lys Phe Lys Val Ala Leu Phe Ser Arg Leu Val Asn
                325                 330                 335

Ala Leu Ser Glu Asp Thr Gly His Ser Ser Tyr Pro Ser His Arg Ser
            340                 345                 350

Phe Thr Lys Met Ser Ser Met Asn Glu Arg Thr Ser Met Asn Glu Arg
        355                 360                 365

Glu Thr Gly Met Leu
    370

<210>  2
<211>  18
<212>  PRT
<213>  artificial sequence

<220>
<223>  Synthetic sequence

<400>  2

Glu Leu His His Thr Ala Met Pro Gly Ser Val Phe Ser Leu Gly Leu
1               5                   10                  15

Pro Leu

<210>  3
<211>  33
<212>  PRT
<213>  artificial sequence

<220>
<223>  Synthetic sequence

<400>  3

Pro Tyr His Thr Leu Asn Leu Leu Glu Leu His His Thr Ala Met Pro
1               5                   10                  15

Gly Ser Val Phe Ser Leu Gly Leu Pro Leu Ala Thr Ala Leu Ala Ile
            20                  25                  30

Ala

<210>  4
<211>  5
<212>  PRT
<213>  artificial sequence

<220>
<223>  Synthetic sequence

<400>  4

Ala Met Pro Gly Ser
1               5

<210>  5
<211>  119
<212>  PRT
<213>  artificial sequence

<220>
<223>  Synthetic sequence

<400>  5

Glu Val Gln Leu Val Ala Ser Gly Gly Gly Leu Val Gln Pro Gly Gly
1               5                   10                  15

Ser Leu Lys Leu Ser Cys Ala Ala Ser Gly Phe Thr Phe Ser Ser Tyr
            20                  25                  30

Gly Met Ser Trp Val Arg Gln Thr Pro Asp Arg Arg Leu Glu Leu Val
        35                  40                  45

Ala Thr Ile Asn Arg Tyr Gly Gly Ser Thr Tyr Tyr Pro Asp Ser Val
    50                  55                  60

Lys Gly Arg Phe Thr Ile Ser Arg Asp Asn Ala Lys Asn Thr Leu Tyr
65                  70                  75                  80

Leu Gln Met Ser Ser Leu Lys Ser Glu Asp Thr Ala Met Tyr Tyr Cys
                85                  90                  95

Pro Arg Leu Ile Tyr Tyr Gly Asn Glu Gly Asp Ser Trp Gly Gln Gly
            100                 105                 110

Thr Thr Leu Thr Val Ser Ser
        115

<210> 6
<211> 108
<212> PRT
<213> artificial sequence

<220>
<223> Synthetic sequence

<400> 6

Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Val Ser Phe Met
            20                  25                  30

His Trp Tyr Gln Gln Lys Ser Gly Thr Ser Pro Lys Arg Trp Ile Tyr
        35                  40                  45

Asp Thr Thr Lys Leu Thr Ser Gly Val Pro Ala Arg Phe Ser Gly Ser
    50                  55                  60

Gly Ser Gly Thr Phe Tyr Ser Leu Thr Ile Ser Ser Met Glu Ala Glu
65                  70                  75                  80

Asp Ala Ala Thr Tyr Tyr Cys Gln Gln Trp Asn Ser Lys Pro Pro Leu
                85                  90                  95

Thr Phe Gly Pro Gly Thr Lys Leu Glu Leu Lys Arg
            100                 105

<210>  7
<211>  119
<212>  PRT
<213>  artificial sequence

<220>
<223>  Synthetic sequence

<400>  7

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Gly Met Ser Trp Val Arg Gln Ala Pro Gly Lys Gly Leu Glu Leu Val
        35                  40                  45

Ala Thr Ile Asn Arg Tyr Gly Gly Ser Thr Tyr Tyr Pro Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Pro Arg Leu Ile Tyr Tyr Gly Asn Glu Gly Asp Ser Trp Gly Gln Gly
            100                 105                 110

Thr Leu Val Thr Val Ser Ser
        115

<210>  8
<211>  107
<212>  PRT
<213>  artificial sequence

<220>
<223>  Synthetic sequence

<400>  8

Ala Ile Gln Leu Thr Gln Ser Pro Ser Ser Leu Ser Ala Ser Val Gly
1               5                   10                  15

Asp Arg Val Thr Ile Thr Cys Ser Ala Ser Ser Ser Val Ser Phe Met
            20                  25                  30

His Trp Tyr Gln Gln Lys Pro Gly Lys Ala Pro Lys Arg Trp Ile Tyr
        35                  40                  45

Asp Thr Thr Lys Leu Thr Ser Gly Val Pro Ser Arg Phe Ser Gly Ser
    50                  55                  60

Gly Ser Gly Thr Asp Tyr Thr Leu Thr Ile Ser Ser Leu Gln Pro Glu
65                  70                  75                  80

Asp Phe Ala Thr Tyr Tyr Cys Gln Gln Trp Asn Ser Lys Pro Pro Leu
                85                  90                  95

Thr Phe Gly Gly Gly Thr Lys Val Glu Ile Lys
            100                 105

<210>  9
<211>  106
<212>  PRT
<213>  artificial sequence

<220>
<223>  Synthetic sequence

<400>  9

Thr Val Ala Ala Pro Ser Val Phe Ile Phe Pro Pro Ser Asp Glu Gln
1               5                   10                  15

Leu Lys Ser Gly Thr Ala Ser Val Val Cys Leu Leu Asn Asn Phe Tyr
            20                  25                  30

Pro Arg Glu Ala Lys Val Gln Trp Lys Val Asp Asn Ala Leu Gln Ser
        35                  40                  45

Gly Asn Ser Gln Glu Ser Val Thr Glu Gln Asp Ser Lys Asp Ser Thr
    50                  55                  60

Tyr Ser Leu Ser Ser Thr Leu Thr Leu Ser Lys Ala Asp Tyr Glu Lys
65                  70                  75                  80

His Lys Val Tyr Ala Cys Glu Val Thr His Gln Gly Leu Ser Ser Pro
                85                  90                  95

Val Thr Lys Ser Phe Asn Arg Gly Glu Cys
            100                 105

<210>  10
<211>  330
<212>  PRT
<213>  artificial sequence

<220>
<223>  Synthetic sequence

<400>  10

<210>  11
<211>  326
<212>  PRT
<213>  artificial sequence

<220>
<223>  Synthetic sequence

<400>  11

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val Glu Arg Lys Cys Cys Val Glu Cys Pro Pro Cys Pro Ala Pro
            100                 105                 110

Pro Val Ala Gly Pro Ser Val Phe Leu Phe Pro Pro Lys Pro Lys Asp
        115                 120                 125

Thr Leu Met Ile Ser Arg Thr Pro Glu Val Thr Cys Val Val Val Asp
    130                 135                 140

Val Ser His Glu Asp Pro Glu Val Gln Phe Asn Trp Tyr Val Asp Gly
145                 150                 155                 160

Val Glu Val His Asn Ala Lys Thr Lys Pro Arg Glu Glu Gln Phe Asn
                165                 170                 175

Ser Thr Phe Arg Val Val Ser Val Leu Thr Val Val His Gln Asp Trp
            180                 185                 190

Leu Asn Gly Lys Glu Tyr Lys Cys Lys Val Ser Asn Lys Gly Leu Pro
        195                 200                 205

Ala Pro Ile Glu Lys Thr Ile Ser Lys Thr Lys Gly Gln Pro Arg Glu
    210                 215                 220

Pro Gln Val Tyr Thr Leu Pro Pro Ser Arg Glu Glu Met Thr Lys Asn
225                 230                 235                 240

Gln Val Ser Leu Thr Cys Leu Val Lys Gly Phe Tyr Pro Ser Asp Ile
                245                 250                 255

Ser Val Glu Trp Glu Ser Asn Gly Gln Pro Glu Asn Asn Tyr Lys Thr
            260                 265                 270

Thr Pro Pro Met Leu Asp Ser Asp Gly Ser Phe Phe Leu Tyr Ser Lys
        275                 280                 285

Leu Thr Val Asp Lys Ser Arg Trp Gln Gln Gly Asn Val Phe Ser Cys
    290                 295                 300

Ser Val Met His Glu Ala Leu His Asn His Tyr Thr Gln Lys Ser Leu
305                 310                 315                 320

Ser Leu Ser Pro Gly Lys
                325

<210> 12
<211> 327
<212> PRT
<213> artificial sequence

<220>
<223>  Synthetic sequence

<400>  12

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Ser Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Leu Gly Lys
                325

<210>  13
<211>  330
<212>  PRT
<213>  artificial sequence

<220>
<223>  Synthetic sequence

<400>  13

Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Lys Val Glu Pro Lys Ser Cys Asp Lys Thr His Thr Cys Pro Pro Cys
            100                 105                 110

Pro Ala Pro Glu Leu Leu Gly Gly Pro Ser Val Phe Leu Phe Pro Pro
        115                 120                 125

Lys Pro Lys Asp Thr Leu Met Ile Ser Arg Thr Pro Glu Val Thr Cys
    130                 135                 140

Val Val Val Asp Val Ser His Glu Asp Pro Glu Val Lys Phe Asn Trp
145                 150                 155                 160

Tyr Val Asp Gly Val Glu Val His Asn Ala Lys Thr Lys Pro Arg Glu
                165                 170                 175

Glu Gln Tyr Asn Ser Thr Tyr Arg Val Val Ser Val Leu Thr Val Leu
            180                 185                 190

His Gln Asp Trp Leu Asn Gly Lys Glu Tyr Lys Cys Lys Val Ser Asn
        195                 200                 205

Lys Ala Leu Pro Ala Pro Ile Ala Lys Thr Ile Ser Lys Ala Lys Gly
    210                 215                 220

Gln Pro Arg Glu Pro Gln Val Tyr Thr Leu Pro Pro Ser Arg Glu Glu
225                 230                 235                 240

Met Thr Lys Asn Gln Val Ser Leu Thr Cys Leu Val Lys Gly Phe Tyr
                245                 250                 255

Pro Ser Asp Ile Ala Val Glu Trp Glu Ser Asn Gly Gln Pro Glu Asn
            260                 265                 270

Asn Tyr Lys Thr Thr Pro Pro Val Leu Asp Ser Asp Gly Ser Phe Phe
        275                 280                 285

Leu Tyr Ser Lys Leu Thr Val Asp Lys Ser Arg Trp Gln Gln Gly Asn
    290                 295                 300

Val Phe Ser Cys Ser Val Met His Glu Ala Leu His Asn His Tyr Thr
305                 310                 315                 320

Gln Lys Ser Leu Ser Leu Ser Pro Gly Lys
                325                 330

<210>  14
<211>  327
<212>  PRT
<213>  artificial sequence

<220>

<223>  Synthetic sequence

<400>  14

Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Lys Thr
65                  70                  75                  80

Tyr Thr Cys Asn Val Asp His Lys Pro Ser Asn Thr Lys Val Asp Lys
                85                  90                  95

Arg Val Glu Ser Lys Tyr Gly Pro Pro Cys Pro Pro Cys Pro Ala Pro
            100                 105                 110

Glu Phe Leu Gly Gly Pro Ser Val Phe Leu Phe Pro Pro Lys Pro Lys
        115                 120                 125

Asp Thr Leu Met Ile Ser Arg Thr Pro Glu Val Thr Cys Val Val Val
    130                 135                 140

Asp Val Ser Gln Glu Asp Pro Glu Val Gln Phe Asn Trp Tyr Val Asp
145                 150                 155                 160

Gly Val Glu Val His Asn Ala Lys Thr Lys Pro Arg Glu Glu Gln Phe
                165                 170                 175

Asn Ser Thr Tyr Arg Val Val Ser Val Leu Thr Val Leu His Gln Asp
            180                 185                 190

Trp Leu Asn Gly Lys Glu Tyr Lys Cys Lys Val Ser Asn Lys Gly Leu
        195                 200                 205

Pro Ser Ser Ile Glu Lys Thr Ile Ser Lys Ala Lys Gly Gln Pro Arg
    210                 215                 220

Glu Pro Gln Val Tyr Thr Leu Pro Pro Ser Gln Glu Glu Met Thr Lys
225                 230                 235                 240

Asn Gln Val Ser Leu Thr Cys Leu Val Lys Gly Phe Tyr Pro Ser Asp
                245                 250                 255

Ile Ala Val Glu Trp Glu Ser Asn Gly Gln Pro Glu Asn Asn Tyr Lys
            260                 265                 270

Thr Thr Pro Pro Val Leu Asp Ser Asp Gly Ser Phe Phe Leu Tyr Ser
        275                 280                 285

Arg Leu Thr Val Asp Lys Ser Arg Trp Gln Glu Gly Asn Val Phe Ser
    290                 295                 300

Cys Ser Val Met His Glu Ala Leu His Asn His Tyr Thr Gln Lys Ser
305                 310                 315                 320

Leu Ser Leu Ser Pro Gly Lys
                325